# EUROPEAN PATENT APPLICATION

(11) **EP 3 467 099 A1**
(43) Date of publication of application: **10.04.2019**
(21) Application number: 17194981.1
(22) Date of filing: 05.10.2017
(51) Int. Cl.: C12N 1/21, C07K 14/34, C12P 13/06, C12P 13/08, C12P 13/10, C12P 13/24

(54) **METHOD FOR THE FERMENTATIVE PRODUCTION OF L-AMINO ACIDS**

(71) Applicant: Evonik Degussa GmbH, 45128 Essen (DE)
(72) Inventor: BEKEL, Thomas, 33790 Halle (Westf.) (DE); THIERBACH, Georg, Dr., 33613 Bielefeld (DE); VOSS, Kornelia, Dr., 33790 Halle (DE)

(57) **Abstract**

The present invention makes available novel bacteria of the genus *Corynebacterium,* preferably of the species *Corynebacterium glutamicum,* in which a polypeptide conferring resistance to lincosamides, selected from lincomycin and clindamycin, encoded by an ImrB gene is modified by eliminating or switching off said polypeptide, and a method for producing an L-amino acid selected from L-lysine, L-valine, L-threonine, L-isoleucine, L-histidine, L-proline and L-ornithine by using such novel bacteria.

## Description

L-Amino acids are used in human medicine, in the pharmaceutical industry, in the food industry and particularly in nutrition of animals.

It is known that L-amino acids such as, for example, L-lysine, are produced by fermentation of strains of the genus *Corynebacterium,* in particular *Corynebacterium glutamicum.* Because of the great economic importance, work is continually being done on improving the production methods. Improvements may relate to the fermentation technology such as e. g. stirring and supplying oxygen, or to the composition of the nutrient media e.g. the sugar concentration during fermentation, or to the processing of the fermentation broth to a suitable product form by e. g. drying and granulating the fermentation broth or ion exchange chromatography or may relate to the intrinsic performance properties of the microorganism itself.

The methods used for improving the performance properties of these microorganisms are those of mutagenesis, selection and screening of mutants. The strains obtained in this way are resistant to anti-metabolites or are auxotrophic for metabolites of regulatory importance, and produce L-amino acids. A well-known anti-metabolite is the L-lysine analogue S-(2-aminoethyl)-L-cysteine (see e.g. Tosaka et al.: Agricultural and Biological Chemistry 42(4), 745-752, (1978)).
Methods of recombinant DNA technology have likewise been used for a number of years for strain improvement of L-amino acid-producing strains of the genus *Corynebacterium,* in particular *Corynebacterium glutamicum,* by modifying, i.e. enhancing or attenuating, individual L-amino acid biosynthesis genes and investigating the effect on L-amino acid production.
The nucleotide sequences of the chromosomes of various bacteria or strains resp. of the genus *Corynebacterium* and of the species *Corynebacterium glutamicum,* and their analysis have been disclosed. This information is available at publicly accessible data bases and may be used for strain development purposes. One such data base is the GenBank data base of the NCBI (National Center for Biotechnology Information, U.S. National Library of Medicine 8600 Rockville Pike, Bethesda MD, 20894 USA).

During the annotation procedure for a sequenced chromosome of an organism identified structures such as e. g. genes or coding sequences are furnished with a unique identifier called locus_tag by the supplier of the information to the data base.

The nucleotide sequence of the *Corynebacterium glutamicum* ATCC13032 chromosome and its analysis was described by Ikeda and Nakagawa (Applied Microbiology and Biotechnology 62, 99-109(2003)) and in EP 1 108 790. It is available at the NCBI under accession number NC_003450. Locus_tag NCgl2592 identifies a sequence coding for a "major facilitator superfamily permease". The nucleotide sequence of the *Corynebacterium glutamicum* R chromosome and its analysis was described by Yukawa et al. (Microbiology 153(4):1042-1058 (2007)). It is available at the NCBI under accession number AP009044. Locus_tag cgR_2586 identifies a sequence coding for a hypothetical protein.

Lv et al. (Journal of Bacteriology 194(3), 742-743 (2012) describe the sequencing of the chromosome of *Corynebacterium glutamicum* ATCC14067, a strain formerly referred to as *Brevibacterium flavum.* It is available at the NCBI under accession number AGQQ02000001 and AGQQ02000002. Locus_tag KIQ_001365 identifies a sequence coding for an "MFS transporter permease".

The nucleotide sequence of the chromosome of *Corynebacterium glutamicum* ATCC13869, a strain formerly referred to as *Brevibacterium lactofermentum,* and its analysis were disclosed by Chen et al. at the NCBI under accession number NZ_CP016335. Locus_tag BBD29_13115 identifies a sequence coding for an "MFS transporter permease".

Kim et al. (Molecules and Cells 12(1), 112-116 (2001)) identified the *ImrB* gene of *Corynebacterium glutamicum,* which encodes a protein conferring resistance to lincosamides, such as lincomycin and clindamycin. The strain of *Corynebacterium glutamicum* used by the authors is referred to as ASO19E12. Strain ASO19E12 originates from ATCC13059 (see: Follettie et al; Journal of Bacteriology 175(13), 4096-4103 (1993)). Kim et al suggested that the resistance to lincomycin is an energy dependent efflux system. The nucleotide sequence of said *ImrB* gene and the encoded amino acid sequence is available at the GenBank data base of the NCBI under accession number AF237667.

Nakagawa discloses under GenBank accession number BAC00079 the encoded amino acid sequence of a protein defined as "Permeases of the major facilitator superfamily" of *Corynebacterium glutamicum* of ATCC13032.

Nishio et al. disclose under GenBank accession number BAV24312 the encoded amino acid sequence of a protein defined as "lincomycin resistance protein ImrB" of *Corynebacterium glutamicum* ssp. *lactofermentum* strain AJ1511.

Chen et al. disclose under GenBank accession number ANU34602 the encoded amino acid sequence of a protein defined as "MFS transporter permease" of *Corynebacterium glutamicum* ATCC 13869. This strain was formerly referred to as *Brevibacterium lactofermentum.*

Yukagawa et al. disclose under Genbank accession number BAF55600 the encoded amino acid sequence of a hypothetical protein belonging to the major facilitator superfamily of *Corynebacterium glutamicum* R.

Lv et al. disclose under GenBank accession number KEI24239 the encoded amino acid sequence of a protein defined as "MFS transporter permease" of *Corynebacterium glutamicum* ATCC 14067. This strain was formerly referred to as *Brevibacterium flavum.*

WO2001000804 discloses various genes encoding stress, resistance and tolerance proteins from *Corynebacterium glutamicum* ATCC 13032 and their use for the modulation of production of fine chemicals. The *ImrB* gene and the encoded polypeptide are disclosed under identifier RXA01524 and SEQ ID NO: 1 and 2 of said application. The application suggests to use drug or antibiotic resistance genes, e.g. the *ImrB* gene, as a tool for the discovery of new antibiotics. It is further suggested to express or overexpress said gene in a suitable host to create an organism, which may be used to screen for novel antibiotic compounds (see page 54 and 95 - 97 of WO2001000804).

Object of the present invention is to provide new measures for the fermentative production of L-amino acids selected from L-lysine, L-valine, L-threonine L-isoleucine, L-histidine, L-proline and L-ornithine by bacteria of the genus *Corynebacterium,* preferably of the species *Corynebacterium glutamicum.*

To achieve the object outlined above the present invention makes available novel bacteria of the genus *Corynebacterium,* preferably of the species *Corynebacterium glutamicum,* having the ability to excrete an L-amino acid selected from L-lysine, L-valine, L-threonine, L-isoleucine, L-histidine, L-proline and L-ornithine, preferably L-lysine, in which a polypeptide conferring resistance to lincosamides, selected from lincomycin and clindamycin, encoded by an *ImrB* gene is modified by eliminating or switching off resp. said polypeptide.

The present invention further makes available a methods for producing said L-amino acids by using said bacteria in a fermentative process and for the manufacturing of a product containing said L-amino acids from the fermentation broth.

The objects underlying the present invention were solved by the means as written in the claims. Accordingly the present invention provides the following:
An isolated bacterium of the genus *Corynebacterium,* preferably of the species *Corynebacterium glutamicum,* having the ability to excrete an L-amino acid selected from L-lysine, L-valine, L-threonine, L-isoleucine, L-histidine, L-proline and L-ornithine, preferably L-lysine, in which an encoded polypeptide having the activity of conferring resistance to lincosamides, selected from lincomycin and clindamycin, preferably lincomycin, and comprising an amino acid sequence, which is at least 90 %, at least 95 %, particularly preferred at least 96 % very particularly preferred at least 97 %, at least 98 % or at least 99 % or 100 %, preferably 99,8 % identical to the amino acid sequence of SEQ ID NO:8, is modified by eliminating said polypeptide from the *Corynebacterium.*

It was found that the bacteria modified according to the invention excreted L-amino acids selected from L-lysine, L-valine, L-threonine L-isoleucine, L-histidine, L-proline and L-ornithine, preferably L-lysine, into a suitable medium under suitable fermentation conditions in an increased manner with respect to one or more parameters selected from product concentration (i.e. amount of L-amino acid produced per volume, or mass unit resp., of medium/fermentation broth (e.g. g/l or g/kg)), product yield (i.e. amount of L-amino acid produced per carbon source consumed (e.g. g/g or kg/kg)), product formation rate (i.e. amount of L-amino acid produced per volume, or mass unit resp., of medium/fermentation broth and unit of time (e.g. g/l x h or g/kg x h)), and specific product formation rate (i.e. amount of L-amino acid produced per unit of time and mass unit of the producer (e.g. g/h x g dry mass)) as compared to the unmodified bacterium.

It is obvious that a higher product concentration facilitates product manufacturing e. g. purification and isolation. An increased product yield reduces the amount of raw material required. An increased product formation rate reduces the time required for a fermentation run thus increasing the availability of a given fermenter.

The bacteria according to the invention thus contribute to the improvement of technical and economic aspects of the manufacturing of L-amino acids selected from L-lysine, L-valine, L-threonine, L-isoleucine, L-histidine, L-proline and L-ornithine.

The term L-amino acids, where mentioned herein, in particular in the context of product formation, also comprises their ionic forms and salts, for example L-lysine mono hydrochloride or L-lysine sulfate in the case of the L-amino acid L-lysine.

For practicing the present invention bacteria of the genus *Corynebacterium* are used. A description of the genus *Corynebacterium* and the species comprised by this genus can be found in the article *"*Corynebacterium" by K. A. Bernard and G. Funke in Bergey's Manual of Systematics of Archaea and Bacteria (Bergey's Manual Trust, 2012).

Within the genus *Corynebacterium* the species *Corynebacterium glutamicum* is preferred.

Suitable strains of *Corynebacterium glutamicum* are wild strains of this species for example strains ATCC13032, ATCC14067 and ATCC13869, and L-amino acid excreting strains obtained from these wild strains, preferably L-amino acid excreting strains obtained from these wild strains.

Strain ATCC13032 (also available as DSM20300) is the taxonomic type strain of the species *Corynebacterium glutamicum.* Strain ATCC14067 (also available as DSM20411) is also known under the outdated designation *Brevibacterium flavum.* Strain ATCC13869 (also available as DSM1412) is also known under the outdated designation *Brevibacterium lactofermentum.* A taxonomic study of this group of bacteria based on DNA-DNA hybridization was done by Liebl et al. (International Journal of Systematic Bacteriology 41(2), 255-260, 1991). A comparative analysis of various strains of the species *Corynebacterium glutamicum* based on genome sequence analysis was provided by Yang and Yang (BMC Genomics 18(1):940).

A multitude of L-amino acid excreting strains of the genus *Corynebacterium* were obtained in the art during the past decades starting from strains such as ATCC13032, ATCC14067, ATCC13869 and the like. They were obtained as a result of strain development programs using inter alia methods like classical mutagenesis, selection for antimetabolite resistance as well as amplification and promotor modification of genes of the biosynthetic pathway of the L-amino acid in question by genetic engineering methods. Summaries may be found in L. Eggeling and M. Bott (Handbook of Corynebacterium glutamicum, CRC Press, 2005) or H. Yukawa and M. Inui (Corynebacterium glutamicum Biology and Biotechnolgy, Springer Verlag, 2013).

L-lysine excreting strains of the species *Corynebacterium glutamicum* are widely known in the art and can be used for the purpose of the present invention. For example Blombach et al (Applied and Environmental Microbiology 75(2), 419-427, 2009) describe strain DM1933, which is deposited under accession DSM25442 according to the Budapest treaty. Strain DM1933 was obtained from ATCC13032 by several steps of strain development. Other L-lysine excreting *Corynebacterium glutamicum* strains are e. g. described in WO2008033001 and EP0841395.

L-lysine excreting strains of the species *Corynebacterium glutamicum* typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild strains like for example ATCC13032, ATCC14067 and ATCC13869. The EC number for aspartokinase is EC 2.7.2.4. Descriptions of polynucleotides of *Corynebacterium glutamicum* encoding a feedback resistant aspartokinase polypeptide variant are for example given in US5688671, US6844176 and US6893848. A summarizing list can be found inter alia in WO2009141330. The symbol used in the art for a gene coding for an aspartokinase polypeptide is *lysC.* In case the gene codes for a feedback resistant polypeptide variant the art typically uses symbols like *lysC^{fbr}* with fbr indicating feedback resistance.

Accordingly said L-lysine excreting strains of the species *Corynebacterium glutamicum* used for the measures of the present invention preferably contain at least (≥) one copy of a polynucleotide coding for a feedback resistant aspartokinase polypeptide.

SEQ ID NO:15 shows the nucleotide sequence of the coding sequence of the aspartokinase polypeptide of strain ATCC13032 and SEQ ID NO: 16 the amino acid sequence of the encoded polypeptide. It is known in the art (see US6893848) that exchange of the amino acid Thr at position 311 of SEQ ID NO:16 for IIe imparts the enzyme feedback resistance to inhibition by mixtures of L-lysine and L-threonine.

Accordingly it is preferred that the amino acid sequence of said feedback resistant aspartokinase polypeptide comprises the amino acid sequence of SEQ ID NO: 16 containing isoleucine at position 311.

Said amino exchange can be achieved by exchanging the nucleobase cytosine (c) at position 932 of SEQ ID NO:15 to give thymine (t). The acc codon for threonine is thus altered to the atc codon for isoleucine.

It is further known in the art that exchange of the gtg start codon of the coding sequence for the aspartokinase polypeptide for atg enhances expression of the polypeptide (see e.g. EP2796555). Accordingly it is preferred that the sequence coding for a feedback resistant aspartokinase polypeptide begins with an atg start codon.

Summaries concerning the breeding of L-lysine excreting strains of *Corynebacterium glutamicum* may be found inter alia in L. Eggeling and M. Bott (Handbook of Corynebacterium glutamicum, CRC Press, 2005), V. F. Wendisch (Amino Acid Biosynthesis - Pathways, Regulation and Metabolic Engineering, Springer Verlag, 2007), H. Yukawa and M. Inui (Corynebacterium glutamicum Biology and Biotechnolgy, Springer Verlag, 2013), and Eggeling and Bott (Applied Microbiology and Biotechnology 99 (9), 3387-3394, 2015).

L-threonine excreting strains of the species *Corynebacterium glutamicum* are known in the art and can be used for the purpose of the present invention. For example EP0385940 describes strain DM368-2, which is deposited under DSM5399.

L-valine excreting strains of the species *Corynebacterium glutamicum* are known in the art and can be used for the purpose of the present invention. For example US5188948 describes strain AJ12341, which is deposited under FERM BP-1763 and EP2811028 describes strain ATCC14067_PprpD2-ilvBN.

L-isoleucine excreting strains of the species *Corynebacterium glutamicum* are known in the art and can be used for the purpose of the present invention. For example US4656135 describes strain AJ12152, which is deposited under Ferm BP-760.

L-histidine excreting strains of the species *Corynebacterium glutamicum* are known in the art, for example in US4495283, and can be used for the purpose of the present invention.

L-proline excreting strains of the species *Corynebacterium glutamicum* are known in the art and can be used for the purpose of the present invention. For example EP1828384 describes an L-proline excreting *Corynebacterium glutamicum* strain comprising a polypeptide having γ-glutamyl kinase activity which contains at position 149 of the encoded amino acid sequence L-aspartic acid.

L-ornithine excreting strains of the species *Corynebacterium glutamicum* are known in the art and can be used for the purpose of the present invention. For example EP2553113 describes L-ornithine excreting *Corynebacterium glutamicum* strain ATCC13032_Delta_argFRGH and transformants derived from the strain.

In case a wild strain, e.g. ATCC13032, ATCC13869 or ATCC14067, is in a first step subjected to the measures of the present invention the resulting strain is in a second step subjected to a strain development program targeted at the desired L-amino acid to obtain a bacterium according to the present invention.

The term DSM denotes the depository Deutsche Sammlung für Mikroorganismen und Zellkulturen located in Braunschweig, Germany. The term ATCC denotes the depository American Type Culure Collection located in Manasass, Virginia, US. The term FERM denotes the depository National Institute of Technology and Evaluation (NITE) located in Tokyo, Japan. Two other well-known depositories are KCCM and NRRL. The term KCCM denotes the depository Korean Culture Center of Microorganisms located in Seoul, Korea. The term NRRL denotes the depository Agricultural Research Service Culture Collection located in Peoria, Illinois, US.

Details regarding the biochemistry and chemical structure of polynucleotides and polypeptides as present in living things such as bacteria like *Corynebacterium* or *Escherichia,* for example, can be found inter alia in the text book "Biochemie" by Berg et al. (Spektrum Akademischer Verlag Heidelberg, Berlin, Germany, 2003; ISBN 3-8274-1303-6).

Polynucleotides consisting of deoxyribonucleotide monomers containing the nucleobases or bases resp. adenine (a), guanine (g), cytosine (c) and thymine (t) are referred to as deoxyribopolynucleotides or deoxyribonucleic acid (DNA). Polynucleotides consisting of ribonucleotide monomers containing the nucleobases or bases resp. adenine (a), guanine (g), cytosine (c) and uracil (u) are referred to as ribo-polynucleotides or ribonucleic acid (RNA). The monomers in said polynucleotides are covalently linked to one another by a 3',5'-phosphodiester bond. By convention single strand polynucleotides are written from 5'- to 3'-direction. Accordingly a polynucleotide has a 5'-end and 3'-end. The order of the nucleotide monomers in the polynucleotide is commonly referred to as nucleotide sequence. Accordingly a polynucleotide is characterized by its nucleotide sequence. For the purpose of this invention deoxyribopolynucleotides are preferred. In bacteria, for example *Corynebacterium* or *Escherichia,* the DNA is typically present in double stranded form. Accordingly the length of a DNA molecule is typically given in base pairs (bp). The nucleotide sequence coding for a specific polypeptide is called coding sequence (cds).

A gene from a chemical point of view is a polynucleotide, preferably a deoxyribopolynucleotide.

The term gene refers to a polynucleotide comprising a nucleotide sequence coding for a specific polypeptide (coding sequence) and the adjoining stop codon. In a broader sense the term includes regulatory sequences preceding and following the coding sequence. The preceding sequence is located at the 5'-end of the coding sequence and is also referred to as upstream sequence. A promotor is an example of a regulatory sequence located 5' to the coding sequence. The sequence following the coding sequence is located at its 3'-end and also referred to as downstream sequence. A transcriptional terminator is an example of a regulatory sequence located 3' to the coding sequence.

Polypeptides consist of L-amino acid monomers joined by peptide bonds. For abbreviation of L-amino acids the one letter code and three letter code of IUPAC (International Union of Pure and Applied Chemistry) is used. Due to the nature of polypeptide biosynthesis polypeptides have an amino terminal end and a carboxyl terminal end also referred to as N-terminal end and C-terminal end. The order of the L-amino acids or L-amino acid residues resp. in the polypeptide is commonly referred to as amino acid sequence. Polypeptides are also referred to as proteins. Further it is known in the art that the start codon or initiation codon resp. gtg of a coding sequence as well as atg encodes the amino acid methionine.

Lincomycin is a lincosamide antibiotic produced by *Streptomyces lincolnensis.* The CAS (Chemical Abstract Service) registry number is 154-21-2. The CAS registry number of the corresponding HCI salt is 859-18-7.

Clindamycin is a chlorinated derivative of lincomycin. The CAS registry number is 18323-44-9. The CAS registry number of the corresponding HCI salt is 21462-39-5.

Summaries concerning antibiotics may be found inter alia in the text book of Jason C. Gallagher and Conan MacDougall (Antibiotics Simplified, 2nd edition, Jones & Bartlett Learning, 2012). It is known in the art that *Corynebacterium,* preferably *Corynebacterium glutamicum,* contains a gene in its chromosome encoding a polypeptide conferring resistance to lincosamide antibiotics selected from lincomycin and clindamycin.

Kim et al. (Molecules and Cells 12(1), 112-116 (2001)) identified the gene in *Corynebacterium glutamicum* strain ASO19E12 and referred to it as *ImrB.* Based on amino acid sequence analysis Kim et al. concluded that the gene encodes a membrane protein belonging to the major facilitators family of proteins with 14 transmembrane segments. Kim et al. further suggested that the *ImrB* conferred resistance to lincomycin is an energy dependent efflux system. The amino acid sequence of the encoded polypeptide is available under GenBank accession number AF237667. It is also shown under SEQ ID NO: 1 of the sequence listing.

Nakagawa discloses under GenBank accession number BAC00079 the encoded amino acid sequence of a polypeptide defined as "Permeases of the major facilitator superfamily" of *Corynebacterium glutamicum* of ATCC13032. It is also shown under SEQ ID NO:2 of the sequence listing.

The amino acid sequences of entries AF237667 and BAC00079 were found to be identical over the full length. Said two amino acid sequences were also found to be identical with the amino acid sequence of the protein of ATCC13032 as disclosed in SEQ ID NO:2 of WO2001000804 (see also GenBank accession number CAC26288).

The amino acid sequence of SEQ ID NO:2 of the sequence listing of the present invention is identical with the amino acid sequence of SEQ ID NO:8 of the sequence listing of the present invention.

Furthermore during the work for the present invention the amino acid sequences of the polypeptides encoded by the *ImrB* genes of different strains of the species *Corynebacterium glutamicum* were analyzed.

Nishio et al. disclose under GenBank accession number BAV24312 the encoded amino acid sequence of a protein defined as "lincomycin resistance protein *ImrB"* of a *Corynebacterium glutamicum* ssp. *lactofermentum* strain referred to as AJ1511. It is also shown under SEQ ID NO:3 of the sequence listing.

Chen et al. disclose under GenBank accession number ANU34602 the encoded amino acid sequence of a protein defined as "MFS transporter permease" of *Corynebacterium glutamicum* ATCC13869. It is also shown under SEQ ID NO:4 of the sequence listing. The amino acid sequences of entries ANU34602 and BAV24312 were found to be identical over the full length. When compared to the amino acid sequence of the corresponding polypeptide of ATCC13032 the identity was found to be 99.2 %.

Yukawa et al. disclose under GenBank accession number BAF55600 the encoded amino acid sequence of a "hypothetical protein cgR_2586" from *Corynebacterium glutamicum* R having a region named "MFS". The amino acid sequence disclosed in BAF55600 is also shown under SEQ ID NO:5 of the sequence listing. Its identity to the corresponding amino acid sequence from ATCC13032 was found to be 96.7 %.

The term "MFS" is the abbreviation for "Major Facilitator Superfamily". According to the conserved domain database at the NCBI (see database entry cd06174) the term denotes a large and diverse group of secondary transporters that includes uniporters, symporters, and antiporters, which facilitate the transport across cytoplasmic or internal membranes of a variety of substrates including ions, sugar phosphates, drugs, neurotransmitters, nucleosides, amino acids, and peptides.

Lv et al. disclose under GenBank accession number KEI24239 the encoded amino acid sequence of a protein from *Corynebacterium glutamicum* ATCC14067 defined as "MFS transporter permease". It is also shown under SEQ ID NO:6 of the sequence listing. Its identity to the corresponding amino acid sequence from ATCC13032 was found to be 97.9 %. A summary of the findings is shown in table 1.

**Table 1: Comparison of the encoded amino acid sequences of LmrB polypeptides of various strains of Corynebacterium glutamicum with the encoded amino acid sequence of the LmrB polypeptide of ATCC13032 (GenBank accession number BAC00079) by sequence alignment using Clustal W (Larkin et al.: Clustal W and Clustal X version 2.0. In: Bioinformatics 23, 2947-2948 (2007)). *number of amino acid residues**

| strain | accession number | length* | identical amino acids | % identity |
|---|---|---|---|---|
| ASO19E12 | AF237667 | 481 | 481 | 100.0 |
| AJ1511 | BAV24312 | 481 | 477 | 99.2 |
| ATCC13869 | ANU34602 | 481 | 477 | 99.2 |
| R | BAF55600 | 481 | 465 | 96.7 |
| ATCC14067 | KEI24239 | 481 | 471 | 97.9 |

During the work for the present invention the coding sequence for the *ImrB* gene of *Corynebacterium glutamicum* strain DM1547, a strain obtained from strain ATCC13032, described in EP1239040 and deposited as DSM13994, was analyzed and the amino acid sequence of the encoded polypeptide deduced. Said polypeptide was found to have the amino acid sequence of SEQ ID NO:8, wherein valine is contained at position 104. Its identity to the corresponding amino acid sequence from ATCC13032 is therefore 99.8 %.

Accordingly said polypeptide conferring resistance to lincosamides, selected from lincomycin and clindamycin, preferably lincomycin, comprises an amino acid sequence being at least 90 %, at least 95 %, at least 96 %, at least 97 %, at least 98 %, at least 99 %, or 100 % identical to the amino acid sequence of SEQ ID NO:8. In one specific embodiment, said polypeptide conferring resistance to lincosamides, selected from lincomycin and clindamycin, preferably lincomycin, comprises an amino acid sequence being at least 99.5 %, preferably at least 99.8 % identical to the amino acid sequence of SEQ ID NO:8, e.g. corresponding to an exchange of alanine by valine at position 104 of SEQ ID NO:8.

Said polypeptide is also referred to as LmrB polypeptide herewith.

Teachings for measuring resistance/sensitivity phenotypes can be found inter alia in text books of medical microbiology such as the textbook of H. Brandis, W. Köhler, H.J. Eggers and G. Pulverer (Lehrbuch der Medizinischen Mikrobiologie, 7th edition, Gustav Fischer Verlag, 1994), by Swenson et al. in Manual of Clinical Microbiology (Murry et al. (eds), 1356-1367, American Society for Microbiology, 1995) or by Kim et al..

It is preferred that the amino acid sequence of said encoded LmrB polypeptide has a length of 481 amino acid residues. It is known in the art that the N-terminal amino acid methionine of an encoded polypeptide may be removed by an aminopeptidase during or after translation (Jocelyn E. Krebs, Elliott S. Goldstein and Stephan T. Kilpatrick: Lewin's Genes X, Jones and Bartlett Publishers, US, 2011).

The amino acid sequence of the encoded LmrB polypeptide from ATCC13032 shown in SEQ ID NO:2 is also shown in SEQ ID NO: 8. The nucleotide sequence encoding said polypeptide is shown in SEQ ID NO: 7 positions 1001 to 2443.

During the work for the present invention the coding sequence for the LmrB polypeptide of strain DM1547 described in EP1239040 and deposited as DSM13994 was analyzed. Said coding sequence of strain DM1547 was found to be identical with that of strain ATCC13032 with the exception of position 311. Position 311 of the coding sequence corresponds to position 1311 of SEQ ID NO:7. Position 311 of the coding sequence in DM1547 contains the nucleobase thymine (t) resulting in a gtg codon for valine. Position 311 of the coding sequence in ATCC13032 contains cytosine (c) resulting in a gcg codon for alanine.

Hence the encoded LmrB polypeptide from DM1547 comprises the amino acid shown in SEQ ID NO:8, wherein valine is contained at position 104. The nucleotide sequence encoding said polypeptide is shown in SEQ ID NO: 7 positions 1001 to 2443, wherein thymine (t) is contained at position 1311.

The amino acid sequence of the encoded LmrB polypeptide from ATCC13869 shown in SEQ ID NO:4 is also shown in SEQ ID NO: 10. The nucleotide sequence encoding said polypeptide is shown in SEQ ID NO:9 positions 1001 to 2443.

The amino acid sequence of the encoded LmrB polypeptide from ATCC14067 shown in SEQ ID NO:6 is also shown in SEQ ID NO: 12. The nucleotide sequence encoding said polypeptide is shown in SEQ ID NO:11 positions 1001 to 2443.

During the work for the present invention it was shown that overexpression of the LmrB polypeptide of ATCC14067 in strain ATCC13032 and in the L-lysine producer DM1933 resulted in an increased resistance towards lincomycin.

It is preferred that said LmrB polypeptide comprises the amino acid sequence of SEQ ID NO:8, SEQ ID NO:8, wherein valine is contained at position 104, SEQ ID NO:10 or SEQ ID NO:12 with SEQ ID NO:8 and SEQ ID NO:8, wherein valine is contained at position 104, being particularly preferred.

The nucleotide sequence encoding said LmrB polypeptide is preferably selected from SEQ ID NO:7 positions 1001 to 2443, SEQ ID NO:7 positions 1001 to 2443, wherein thymine (t) is contained at position 1311, SEQ ID NO:9 positions 1001 to 2443 and SEQ ID NO:11 positions 1001 to 2443, with SEQ ID NO:7 positions 1001 to 2443 and SEQ ID NO:7 positions 1001 to 2443, wherein thymine (t) is contained at position 1311, being particularly preferred.

Teachings and information concerning the handling of and experimental work with polynucleotides may be found inter alia in the handbook of J. Sambrook et al. (Molecular Cloning, A Laboratory Manual, Cold Spring Harbor Laboratory Press, 1989), the textbook of C. R. Newton and A. Graham (PCR, Spektrum Akademischer Verlag, 1994) and the handbook of D. Rickwood and B. D. Hames (Gel electrophoresis of nucleic acids, a practical approach, IRL Press, 1982).

For sequence analysis of polynucleotides and polypeptides, e.g. sequence alignments, public software such as the CLC Genomics Workbench (Qiagen, Hilden, Germany) or the program MUSCLE provided by the European Bioinformatics Institute (EMBL-EBI, Hinxton, UK) may also be used.

During the work for the present invention it was found that modifying L-amino acid excreting bacteria of the genus *Corynebacterium,* preferably of the species *Corynebacterium glutamicum,* by eliminating the LmrB polypeptide increased their ability to excrete L-amino acids as compared to the unmodified bacterium.

The skilled artisan is aware of a number of methods of mutagenesis how to achieve said eliminating or switching off resp. of said LmrB polypeptide in the *Corynebacterium.*

The preferred embodiments are explained in more detail below.

In a preferred embodiment said eliminating is achieved by by
a) deleting one or two nucleotides in the part of the coding sequence of said LmrB polypeptide corresponding to amino acids of positions 1 to 258 of the encoded amino acid sequence, or
b) deleting at least the part of the coding sequence of said LmrB polypeptide corresponding to amino acids of positions 210 to 258, positions 175 to 301, positions 119 to 364 or of positions 58 to 439 of the encoded amino acid sequence, particularly preferred deleting at least the complete coding sequence.

As a consequence of said eliminating by deleting according to a) or b), preferably b), novel junction points or junction sites resp. are created in the chromosome of the *Corynebacterium,* preferably *Corynebacterium glutamicum.*

If for example the complete coding sequence is deleted a novel junction point is created in the chromosome of the *Corynebacterium,* preferably *Corynebacterium glutamicum,* which links the first nucleobase of the stop codon adjoining the coding sequence, e.g. the nucleobase at position 2444 of SEQ ID NO:7, SEQ ID NO:9 or SEQ ID NO:11, with the first nucleobase preceding the start codon of the coding sequence, e.g. the nucleobase at position 1000 of SEQ ID NO:7, SEQ ID NO:9 or SEQ ID NO:11.

In a specific embodiment according to b) the nucleotide sequence from positions 989 to 2455 of SEQ ID NO:7, of SEQ ID NO:7, wherein thymine (t) is contained at position 1311, of SEQ ID NO:9 or of SEQ ID NO:11, which comprises the coding sequence of said LmrB polypeptide and the adjoining stop codon, is deleted and the nucleotide sequence gatatc, which is the recognition site for the restriction endonuclease *EcoRV,* inserted into the site of deletion.

Thus a novel junction site is created in the chromosome of the *Corynebacterium glutamicum* characterized by the gatatc nucleotide sequence bridge between the nucleobase at position 988 and the nucleobase at position 2456 of SEQ ID NO:7, SEQ ID NO:9 or SEQ ID NO:11. Strains ATCC13032, DM1547 and ATCC13869 contain at position 988 the nucleobase cytosine (c) as shown in SEQ ID NO:7 and SEQ ID NO:9. Strain ATCC14067 contains at position 988 the nucleobase thymine (t) as shown in SEQ ID NO:11. At position 2456 the three strains contain the nucleobase thymine as shown in SEQ ID NO:7, SEQ ID NO:9 and SEQ ID NO:11. The nucleotide sequence of the novel junction site created including the nucleotide sequences upstream and downstream therefrom are shown in SEQ ID NO:13 and SEQ ID NO:14 and table 2, table 3, table 4, table 5, table 6, table 7 and table 8.

Accordingly in a more specific embodiment of the present invention a deletion in the chromosome of a *Corynebacterium glutamicum,* preferably ATCC13032, ATCC13869, ATCC14067 and L-amino acid excreting strains obtained from these strains, comprising the complete coding sequence for the LmrB polypeptide and the adjoining stop codon accompanied by the insertion of the recognition site for the restriction enzyme *EcoRV* is identified by a nucleotide sequence selected from table 2.

Accordingly in another more specific embodiment of the present invention a deletion in the chromosome of *Corynebacterium glutamicum* strains, preferably ATCC13032, ATCC13869 and L-amino acid excreting strains obtained from these strains, comprising the complete coding sequence for the LmrB polypeptide and the adjoining stop codon accompanied by the insertion of the recognition site for the restriction enzyme *EcoRV* is identified by a nucleotide sequence selected from table 3.

Accordingly in another more specific embodiment of the present invention a deletion in the chromosome of *Corynebacterium glutamicum* strains, preferably ATCC13032, ATCC13869 and L-amino acid excreting strains obtained from these strains, comprising the complete coding sequence for the LmrB polypeptide and the adjoining stop codon accompanied by the insertion of the recognition site for the restriction enzyme *EcoRV* is identified by a nucleotide sequence selected from table 4.

Accordingly in another more specific embodiment of the present invention a deletion in the chromosome of *Corynebacterium glutamicum* strains, preferably ATCC13032, ATCC13869 and L-amino acid excreting strains obtained from these strains, comprising the complete coding sequence for the LmrB polypeptide and the adjoining stop codon accompanied by the insertion of the recognition site for the restriction enzyme *EcoRV* is identified by a nucleotide sequence selected from table 5.

Accordingly in another more specific embodiment of the present invention a deletion in the chromosome of *Corynebacterium glutamicum* strains, preferably ATCC13032, ATCC13869 and L-amino acid excreting strains obtained from these strains, comprising the complete coding sequence for the LmrB polypeptide and the adjoining stop codon accompanied by the insertion of the recognition site for the restriction enzyme *EcoRV* is identified by a nucleotide sequence selected from table 6.

Accordingly in another more specific embodiment of the present invention a deletion in the chromosome of *Corynebacterium glutamicum* strains, preferably ATCC14067 and L-amino acid excreting strains obtained therefrom, comprising the complete coding sequence for the LmrB polypeptide and the adjoining stop codon accompanied by the insertion of the recognition site for the restriction enzyme *EcoRV* is identified by a nucleotide sequence selected from table 7. Accordingly in another more specific embodiment of the present invention a deletion in the chromosome of *Corynebacterium glutamicum* strains, preferably ATCC14067 and L-amino acid excreting strains obtained therefrom, comprising the complete coding sequence for the LmrB polypeptide and the adjoining stop codon accompanied by the insertion of the recognition site for the restriction enzyme *EcoRV* is identified by a nucleotide sequence selected from table 8.

**Table 2: List of nucleotide sequences indicating a deletion in the chromosome of a Corynebacterium glutamicum comprising the coding sequence and the adjoining stop codon of the ImrB gene in accordance with the present invention. The nucleotide sequence for the recognition site for the restriction endonuclease EcoRV extends from position 789 to 794 in SEQ ID NO:13 and SEQ ID NO:14.**

| line | nucleotide sequence | length* |
|---|---|---|
| a | SEQ ID NO:13 positions 789 to 801 | 13 |
| b | SEQ ID NO: 13 positions 789 to 802 | 14 |
| c | SEQ ID NO: 13 positions 789 to 803 | 15 |
| d | SEQ ID NO: 13 positions 789 to 804 | 16 |
| e | SEQ ID NO: 13 positions 789 to 805 | 17 |

| | | |
|---|---|---|
| *length in nucleobases or base pairs resp. | | |

**Table 3: List of nucleotide sequences indicating a deletion in the chromosome of strains of Corynebacterium glutamicum comprising the coding sequence and the adjoining stop codon of the ImrB gene in accordance with the present invention. The nucleotide sequence of the recognition site for the restriction endonuclease EcoRV extends from position 789 to 794 in SEQ ID NO:13.**

| line | nucleotide sequence | length* |
|---|---|---|
| a | SEQ ID NO: 13 positions 788 to 800 | 13 |
| b | SEQ ID NO:13 positions 788 to 801 | 14 |
| c | SEQ ID NO: 13 positions 788 to 802 | 15 |
| d | SEQ ID NO: 13 positions 788 to 803 | 16 |
| e | SEQ ID NO: 13 positions 788 to 804 | 17 |

| | | |
|---|---|---|
| *length in nucleobases or base pairs resp. | | |

**Table 4: List of nucleotide sequences indicating a deletion in the chromosome of strains of Corynebacterium glutamicum comprising the coding sequence and the adjoining stop codon of the ImrB gene in accordance with the present invention. The nucleotide sequence of the recognition site for the restriction endonuclease EcoRV extends from position 789 to 794 in SEQ ID NO:13.**

| line | nucleotide sequence | length* |
|---|---|---|
| a | SEQ ID NO:13 positions 787 to 798 | 12 |
| b | SEQ ID NO: 13 positions 787 to 899 | 13 |
| c | SEQ ID NO: 13 positions 787 to 800 | 14 |
| d | SEQ ID NO:13 positions 787 to 801 | 15 |
| e | SEQ ID NO: 13 positions 787 to 802 | 16 |
| f | SEQ ID NO: 13 positions 787 to 803 | 17 |

| | | |
|---|---|---|
| *length in nucleobases or base pairs resp. | | |

**Table 5: List of nucleotide sequences indicating a deletion in the chromosome of strains of Corynebacterium glutamicum comprising the coding sequence and the adjoining stop codon of the ImrB gene in accordance with the present invention. The nucleotide sequence of the recognition site for the restriction endonuclease EcoRV extends from position 789 to 794 in SEQ ID NO:13.**

| line | nucleotide sequence | length* |
|---|---|---|
| a | SEQ ID NO: 13 positions 784 to 795 | 12 |
| b | SEQ ID NO:13 positions 783 to 795 | 13 |
| c | SEQ ID NO:13 positions 782 to 795 | 14 |
| d | SEQ ID NO:13 positions 781 to 795 | 15 |
| e | SEQ ID NO:13 positions 780 to 795 | 16 |
| f | SEQ ID NO:13 positions 779 to 795 | 17 |

| | | |
|---|---|---|
| *length in nucleobases or base pairs resp. | | |

**Table 6: List of nucleotide sequences indicating a deletion in the chromosome of strains of Corynebacterium glutamicum comprising the coding sequence and the adjoining stop codon of the ImrB gene in accordance with the present invention. The nucleotide sequence of the recognition site for the restriction endonuclease EcoRV extends from position 789 to 794 in SEQ ID NO:13.**

| line | nucleotide sequence | length* |
|---|---|---|
| a | SEQ ID NO:13 positions 784 to 796 | 13 |
| b | SEQ ID NO:13 positions 783 to 796 | 14 |
| c | SEQ ID NO: 13 positions 782 to 796 | 15 |
| d | SEQ ID NO:13 positions 781 to 796 | 16 |
| e | SEQ ID NO:13 positions 780 to 796 | 17 |

| | | |
|---|---|---|
| *length in nucleobases or base pairs resp. | | |

**Table 7: List of nucleotide sequences indicating a deletion in the chromosome of strains of Corynebacterium glutamicum comprising the coding sequence and the adjoining stop codon of the ImrB gene in accordance with the present invention. The nucleotide sequence of the recognition site for the restriction endonuclease EcoRV extends from position 789 to 794 in SEQ ID NO:14.**

| line | nucleotide sequence | length* |
|---|---|---|
| a | SEQ ID NO:14 positions 782 to 795 | 14 |
| b | SEQ ID NO:14 positions 781 to 795 | 15 |
| c | SEQ ID NO:14 positions 780 to 795 | 16 |
| d | SEQ ID NO:14 positions 779 to 795 | 17 |
| e | SEQ ID NO:14 positions 778 to 795 | 18 |

| | | |
|---|---|---|
| *length in nucleobases or base pairs resp. | | |

**Table 8: List of nucleotide sequences indicating a deletion in the chromosome of strains of Corynebacterium glutamicum comprising the coding sequence and the adjoining stop codon of the ImrB gene in accordance with the present invention. The nucleotide sequence of the recognition site for the restriction endonuclease EcoRV extends from position 789 to 794 in SEQ ID NO:14.**

| line | nucleotide sequence | length* |
|---|---|---|
| a | SEQ ID NO:14 positions 788 to 801 | 14 |
| b | SEQ ID NO: 14 positions 788 to 802 | 15 |
| c | SEQ ID NO:14 positions 788 to 803 | 16 |
| d | SEQ ID NO:14 positions 788 to 804 | 17 |

| | | |
|---|---|---|
| *length in nucleobases or base pairs resp. | | |

In another preferred embodiment said eliminating is achieved by
a) inserting one or two nucleotides into the part of the coding sequence of said LmrB polypeptide corresponding to amino acids of positions 1 to 258 of the encoded amino acid sequence,
b) inserting a gene coding for a polypeptide of the biosynthetic pathway of an L-amino acid selected from L-lysine, L-valine, L-threonine, L-isoleucine, L-histidine, L-proline and L-ornithine, preferably L-lysine, into the part of the coding sequence corresponding to amino acids of positions 175 to 258, preferably positions 210 to 258 of the amino acid sequence of said LmrB polypeptide. Said genes coding for a polypeptide of the biosynthetic pathway of L-lysine is preferably selected from the list comprising
   - a gene *pyc* of *Corynebacterium glutamicum* coding for pyruvate carboxylase (EC 6.4.1.1) as e.g. described in WO1999018228 or EP2107128,
   - a gene *aspB* of *Corynebacterium glutamicum* coding for aspartate amino transferase (EC 2.6.1.1) as e.g. described in EP0219027 or WO2008033001,
   - a gene *lysC* of *Corynebacterium glutamicum* coding for an aspartate kinase, preferably feedback resistant aspartate kinase (EC 2.7.2.4), as e.g. described in US6893848,
   - a gene asd of *Corynebacterium glutamicum* coding for aspartate semialdehyde dehydrogenase (EC 1.2.1.11) as e.g. described in EP0387527 or WO2008033001,
   - a gene *dapA* of *Corynebacterium glutamicum* coding for dihydrodipicolinate synthase (EC 4.3.3.7) as e.g. described in EP0197335,
   - a gene *dapB* of *Corynebacterium glutamicum* coding for dihydrodipicolinate reductase (EC 1.17.1.8) as e.g. described in US8637295 or EP0841395,
   - a gene *ddh* of *Corynebacterium glutamicum* coding for diaminopimelate dehydrogenase (EC 1.4.1.16) as e.g. described in EP0811682,
   - a gene *lysE* of *Corynebacterium glutamicum* coding for lysine permease as e.g. described in EP0868527,
   - a gene *dapF* of *Corynebacterium glutamicum* coding for diaminopimelate epimerase as e.g. described in US6670156, and
   - a gene *lysA* of *Corynebacterium glutamicum* coding for diaminopimelate decarboxylase (EC 4.1.1.20) as e.g. described in EP0811682.

In a further preferred embodiment said eliminating is achieved by substituting one or more codons coding for an amino acid of said polypeptide with a taa-, tga- or tag- stop codon, preferably a taa- or tga- stop codon, particularly preferred taa- stop codon, in the part of coding sequence corresponding to amino acids of positions 1 to 258, preferably positions 1 to 20, particularly preferred positions 1 to 2 of the encoded amino acid sequence.

In the expression "one or more codons" the term "more" means any positive integer or natural number resp. up to 258, preferably up to 20, particularly preferred up to 10, more particularly preferred up to 3 or 2, when it relates to a substitution of a codon for an amino acid in the part of coding sequence corresponding to amino acids of positions 1 to 258 of the encoded amino acid sequence.

It means any positive integer or natural number resp. up to 20, preferably up to 10, particularly preferred up to 3 or 2, when it relates to a substitution of a codon for an amino acid in the part of coding sequence corresponding to amino acids of positions 1 to 20 of the encoded amino acid sequence.

It means 2 (two) when it relates to a substitution of a codon for an amino acid in the part of coding sequence corresponding to amino acids of positions 1 to 2 of the encoded amino acid sequence.

In a further preferred embodiment said eliminating is achieved by inserting at least one stop codon, preferably no more than 10 stop codons, selected from taa-, tga- or tag- stop codon, preferably taa- or tga- stop codon, particularly preferred taa- stop codon, between at least one pair of adjacent or neighbouring resp. codons in the part of coding sequence corresponding to amino acids of positions 1 to 258, preferably positions 1 to 20, particularly preferred positions 1 to 2 of the encoded amino acid sequence of said polypeptide.

For example one or two taa- stop codon(s) may be inserted between the codons coding for the amino acids at position 1 and 2 of the amino acid sequence shown in SEQ ID NO:8 or SEQ ID NO:8, wherein valine is contained at position 104, or accordingly between positions 1003 and 1004 of the nucleotide sequence shown in SEQ ID NO:7 or of the nucleotide sequence shown in SEQ ID NO:7, wherein thymine (t) is contained at position 1311.

A common method of mutating genes of *Corynebacterium glutamicum* is the method and of gene replacement described by Schwarzer and Pühler (Bio/Technology 9, 84-87 (1991)) and further elaborated by Schafer et al. (Gene 145, 69-73 (1994)).

Peters-Wendisch et al. (Microbiology 144, 915-927 (1998)) used the gene replacement method to inactivate the *pyc* gene of *Corynebacterium glutamicum* encoding pyruvate carboxylase. Schafer et al. used the method to incorporate a deletion into the *hom-thrB* gene region of *Corynebacterium glutamicum.* In EP1094111 the method was used to incorporate a deletion into the pck gene of *Corynebacterium glutamicum* encoding phosphoenol pyruvate carboxykinase.

In the gene replacement method, a mutation, such as, for example, a deletion, insertion or substitution of at least one nucleobase, is constructed in vitro in the nucleotide sequence of the gene in question. In the context of the present invention the nucleotide sequence of the gene in question (*ImrB* gene) encodes a polypeptide having the activity of conferring resistance to lincosamides as specified in this invention. Examples of such nucleotide sequences are SEQ ID NO:7, SEQ ID NO:7 containing thymine (t) at position 1311, SEQ ID NO:9 and SEQ ID NO: 11 of the sequence listing. In the context of the present invention the mutation is a deletion, insertion or substitution of at least one nucleobase, preferably deletion of at least one nucleobase, located in the coding sequence of said *ImrB* gene.

The mutated nucleotide sequence of the gene in question comprises i) a nucleotide sequence at the 5'-end of the site of mutation, which is also referred to as 5'-flanking sequence or upstream sequence in the art, ii) a nucleotide sequence at the 3'-end of the site of mutation, which is also referred to as 3'-flanking sequence or downstream sequence in the art, and iii) the nucleotide sequence of the site of mutation between i) and ii). In case of a deletion the site of mutation is besides the lack of a specific sequence, also characterized by the flanking sequences forming the novel junction point. In the context of the present invention said deletion preferably concerns the complete coding sequence for the LmrB polypeptide or a part thereof.

For some applications it may be convenient to further incorporate a suitable polynucleotide into said site of mutation. Said suitable polynucleotide may inter alia contain the coding sequence for an enzyme of the biosynthetic pathway of an L-amino acid, e. g. the coding sequence for the enzyme aspartokinase, which is an enzyme of the L-lysine biosynthetic pathway, or the nucleotide sequence of the recognition site for a restriction enzyme useful for further strain improvement.

An example of a mutated nucleotide sequence in the context of the present invention is shown in SEQ ID NO: 13. The 5'-flanking sequence consists of the nucleotide sequence from positions 201 to 988 of SEQ ID NO:7. The 3'-flanking sequence consists of the nucleotide sequence from positions 2456 to 3246 of SEQ ID NO:7. The nucleotide sequence from positions 989 to 2455 of SEQ ID NO:7 comprising the coding sequence of the LmrB polypeptide was removed or deleted resp.. and additionally the nucleotide sequence of the recognition site for the restriction endonuclease EcoRV incorporated as shown from positions 789 to 794 of SEQ ID NO: 13.

Another example of a mutated nucleotide sequence in the context of the present invention is shown in SEQ ID NO:14. The 5'-flanking sequence consists of the nucleotide sequence from positions 201 to 988 of SEQ ID NO:11. The 3'-flanking sequence consists of the nucleotide sequence from positions 2456 to 3246 of SEQ ID NO:11. The nucleotide sequence from positions 989 to 2455 of SEQ ID NO: 11 comprising the coding sequence of the LmrB polypeptide was removed or deleted resp. and additionally the nucleotide sequence of the recognition site for the restriction endonuclease EcoRV incorporated as shown from positions 789 to 794 of SEQ ID NO:14.

The mutated nucleotide sequence constructed is cloned into a plasmid vector that is not capable of autonomous replication in *Corynebacterium glutamicum.* Said plasmid vector comprising said mutated nucleotide sequence is subsequently transferred into the desired strain of *Corynebacterium glutamicum* by transformation or conjugation. After two events of homologous recombination comprising a recombination event within the 5'-flanking sequence provided by the plasmid vector with the homologous sequence of the *Corynebacterium glutamicum* chromosome and a recombination event within the 3'-flanking sequence provided by the plasmid vector with the homologous sequence of the *Corynebacterium glutamicum* chromosome, one effecting integration and one effecting excision of said plasmid vector, the mutation is incorporated in the *Corynebacterium glutamicum* chromosome. Thus the nucleotide sequence of the gene in question contained in the chromosome of said desired strain is replaced by the mutated nucleotide sequence.

An event of homologous recombination may also be referred to as crossing over.

It is preferred that the L-amino acid excreting strains of *Corynebacterium,* preferably *Corynebacterium glutamicum,* of the present invention have the ability to excrete ≥ 0,1 g/l, preferably ≥ 0,25 g/l, particularly preferred ≥ 0,5 g/l of the desired L-amino acid in a suitable medium under suitable conditions.

The invention further provides a fermentative process for producing an L-amino acid selected from L-lysine, L-valine, L-threonine, L-isoleucine, L-histidine, L-proline and L-ornithine, preferably L-lysine, using the *Corynebacterium* of the present invention.

In a fermentative process according to the invention a *Corynebacterium,* preferably *Corynebacterium glutamicum,* modified in accordance with the present invention and having the ability to excrete an L-amino is cultivated in a suitable medium under suitable conditions. Due to said ability to excrete said L-amino acid the concentration of the L-amino acid increases and accumulates in the medium during the fermentative process and the L-amino acid is thus produced.

The fermentative process may be discontinuous process like a batch process or a fed batch process or a continuous process. A summary concerning the general nature of fermentation processes is available in the textbook by H. Chmiel (Bioprozesstechnik, Spektrum Akademischer Verlag, 2011), in the textbook of C. Ratledge and B. Kristiansen (Basic Biotechnology, Cambridge University Press,2006) or in the textbook of V.C. Hass and R. Pörtner (Praxis der Bioprozesstechnik Spektrum Akademischer Verlag, 2011).

A suitable medium used for the production of an L-amino acid by a fermentative process contains a carbon source, a nitrogen source, a phosphorus source, inorganic ions and other organic compounds as required.

Suitable carbon sources include glucose, fructose, sucrose as well as the corresponding raw materials like starch hydrolysate, molasses or high fructose corn syrup.

As nitrogen source organic nitrogen-containing compounds such as peptones, meat extract, soy bean hydrolysates or urea, or inorganic compounds such as ammonium sulphate, ammonium chloride, ammonium phosphate, ammonium carbonate, ammonium nitrate, ammonium gas or aqueous ammonia can be used.
As phosphorus source, phosphoric acid, potassium dihydrogen phosphate or dipotassium hydrogen phosphate or the corresponding sodium-containing salts can be used.

Inorganic ions like potassium, sodium, magnesium, calcium, iron and further trace elements etc. are supplied as salts of sulfuric acid, phosphoric acid or hydrochloric acid.

Other organic compounds means essential growth factors like vitamins e. g. thiamine or biotin or L-amino acids e. g. L-homoserine.

The media components may be added to the culture in form of a single batch or be fed in during the cultivation in a suitable manner.

During the fermentative process the pH of the culture can be controlled by employing basic compounds such as sodium hydroxide, potassium hydroxide, ammonia or aqueous ammonia, or acidic compounds such as phosphoric acid or sulphuric acid in a suitable manner. The pH is generally adjusted to a value of from 6.0 to 8.5, preferably 6.5 to 8.0. To control foaming, it is possible to employ antifoam agents such as, for example, fatty acid polyglycol esters. To maintain the stability of plasmids, it is possible to add to the medium suitable selective substances such as, for example, antibiotics. The fermentative process is preferably carried out under aerobic conditions. In order to maintain these conditions, oxygen or oxygen-containing gas mixtures such as, for example air are introduced into the culture. The fermentative process is carried out, where appropriate, at elevated pressure, for example at an elevated pressure of 0.03 to 0.2 MPa. The temperature of the culture is normally from 25 °C to 40 °C, preferably from 30 °C to 37 °C. In a discontinuous process, the cultivation is continued until an amount of the desired L-amino acid sufficient for being recovered has been formed. The cultivation is then completed. This aim is normally achieved within 10 hours to 160 hours. In continuous processes, longer cultivation times are possible.

Examples of suitable media and culture conditions can be found inter alia in L. Eggeling and M. Bott (Handbook of Corynebacterium glutamicum, CRC Press, 2005) and the patent documents US5770409, US5990350, US 5275940, US5763230 and US6025169.

Thus the fermentative process results in a fermentation broth which contains the desired L-amino acid.

Accordingly a method for the fermentative production of an L-amino acid is provided comprising the steps of
a) cultivating a *Corynebacterium* of the present invention
   in a suitable medium under suitable conditions, and
b) accumulating said L-amino acid in the medium to produce an L-amino acid containing fermentation broth.

A product containing the L-amino acid is then recovered in liquid or solid from the fermentation broth.

A "fermentation broth" means a medium in which a *Corynebacterium* of the invention has been cultivated for a certain time and under certain conditions.

When the fermentative process is completed, the resulting fermentation broth accordingly comprises:
a) the biomass (cell mass) of the *Corynebacterium* of the invention, said biomass having been produced due to propagation of the cells of said *Corynebacterium,*
b) the desired fine chemical accumulated during the fermentative process,
c) the organic by-products accumulated during the fermentative process, and
d) the components of the medium employed which have not been consumed in the fermentative process.

The organic by-products include compounds which may be formed by the *Corynebacterium* of the invention during the fermentative process in addition to production of the desired L-amino acid.

The fermentation broth is removed from the culture vessel or fermentation tank, collected where appropriate, and used for providing a product containing the fine chemical, preferably an L-amino acid-containing product, in liquid or solid form. The expression "recovering the fine chemical-containing product" is also used for this. In the simplest case, the L-amino acid-containing fermentation broth itself, which has been removed from the fermentation tank, constitutes the recovered product.

The fermentation broth can subsequently be subjected to one or more of the measures selected from the group consisting of:
a) partial (>0% to <80%) to complete (100%) or virtually complete ( ≥ 80%, ≥ 90%, ≥ 95%, ≥ 96%, ≥ 97%, ≥ 98%, ≥ 99%) removal of the water,
b) partial (>0% to <80%) to complete (100%) or virtually complete (≥ 80%, ≥ 90%, ≥ 95%, ≥ 96%, ≥ 97%, ≥ 98%, ≥ 99%) removal of the biomass, the latter being optionally inactivated before removal,
c) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the organic by-products formed during the fermentative process, and
d) partial (>0%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the residual components of the medium employed or of the residual input materials resp., which have not been consumed in the fermentative process.

An abundance of technical instructions for measures a), b), c) and d) are available in the art.

Removal of water (measure a)) can be achieved inter alia by evaporation, using e.g. a falling film evaporator, by reverse osmosis or nanofiltration. The concentrates thus obtained can be further worked up by spray drying or spray granulation. It is likewise possible to dry the fermentation broth directly using spray drying or spray granulation.

Removal of the biomass (measure b)) can be achieved inter alia by centrifugation, filtration or decantation or a combination thereof.

Removal of the organic by-products (measure c)) or removal of residual components of the medium (measure d) can be achieved inter alia by chromatography, e.g. ion exchange chromatography, treatment with activated carbon or crystallization. In case the organic by-products or residual components of the medium are present in the fermentation broth as solids they can be removed by measure b).

General instructions on separation, purification and granulation methods can be found inter alia in the book of R. Ghosh "Principles of Bioseperation Engineering" (World Scientific Publishing, 2006), the book of F. J. Dechow "Seperation and Purification Techniques in Biotechnology" (Noyes Publications, 1989), the article "Bioseparation" of Shaeiwitz et al. (Ullmann's Encyclopedia of Industrial Chemistry, Wiley-VCH, 2012) and the book of P. Serno et al. "Granulieren" (Editio Cantor Verlag, 2007).

A downstream processing scheme for L-lysine products can be found in the article "L-lysine Production" of R. Kelle et al. (L. Eggeling and M. Bott (Handbook of Corynebacterium glutamicum, CRC Press, 2005)). US5279744 teaches the manufacturing of a purified L-lysine product by ion exchange chromatography. US4956471 teaches the manufacturing of a purified L-valine product by ion exchange chromatography. US5431933 teaches the manufacturing of dry L-amino acid products, e. g. an L-lysine product or an L-valine product, containing most of the constituents of the fermentation broth.

Thus a concentration or purification of the desired L-amino acid is achieved and a product having the desired content of said L-amino acid is provided.

Analysis of L-amino acids to determine the concentration at one or more time(s) during the fermentation can take place by separating the L-amino acids by means of ion exchange chromatography, preferably cation exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion exchange chromatography can be found in Pickering (LC.GC (Magazine of Chromatographic Science 7(6):484-487 (1989)). It is likewise possible to carry out a pre-column derivatization, for example using ortho-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51:1167-1174 (1979)). Detection is carried out photometrically (absorption, fluorescence). A review regarding amino acid analysis can be found inter alia in the textbook "Bioanalytik" by Lottspeich and Zorbas (Spektrum Akademischer Verlag, Heidelberg, Germany 1998).

## Claims

1. A bacterium of the genus *Corynebacterium* having the ability to excrete an L-amino acid selected from L-lysine, L-valine, L-threonine, L-isoleucine, L-histidine, L-proline and L-ornithine, in which an encoded polypeptide having the activity of conferring resistance to lincosamides, selected from lincomycin and clindamycin, and comprising an amino acid sequence, which is at least 90 % identical to the amino acid sequence of SEQ ID NO:8, is modified by eliminating said polypeptide.

2. The bacterium of claim 1, wherein said *Corynebacterium* belongs to the species *Corynebacterium glutamicum.*

3. The bacterium of claim 1 or 2, wherein said L-amino acid is L-lysine.

4. The bacterium as claimed in any of claims 1 to 3, wherein said polypeptide has the activity of conferring lincomycin resistance to the bacterium.

5. The bacterium as claimed in any of claims 1 to 4, wherein the amino acid sequence of said encoded polypeptide comprises 481 amino acids.

6. The bacterium as claimed in any of claims 1 to 5, wherein said polypeptide comprises an amino acid sequence selected from SEQ ID NO:8 or SEQ ID NO:8 wherein valine is contained at position 104, or SEQ ID NO:10 or SEQ ID NO:12.

7. The bacterium of claim 6, wherein said encoded polypeptide comprises the amino acid sequence of SEQ ID NO:8 or SEQ ID NO:8 wherein valine is contained at position 104.

8. The bacterium of claim 7, wherein said polypeptide is encoded by the nucleotide sequence of SEQ ID NO:7 positions 1001 to 2443 or SEQ ID NO:7 positions 1001 to 2443, wherein thymine (t) is contained at position 1311.

9. The bacterium as claimed in any of claims 1 to 8, wherein said eliminating the activity is achieved by deleting one or two nucleotides in the part of the coding sequence of said polypeptide corresponding to amino acids of positions 1 to 258 of the encoded amino acid sequence.

10. The bacterium as claimed in any of claims 1 to 8, wherein said eliminating the activity is achieved by deleting at least the part of the coding sequence of said polypeptide corresponding to amino acids of positions 210 to 258 of the encoded amino acid sequence.

11. The bacterium of claim 10, wherein at least the complete coding sequence is deleted.

12. The bacterium of claim 11, wherein at least the complete coding sequence and the adjoining stop codon is deleted.

13. The bacterium of claim 12, wherein said deletion is identified by any of the nucleotide sequences shown in table 2, table 3, table 4, table 5, table 6, table 7 or table 8.

14. The bacterium as claimed in any of claims 1 to 8, wherein said eliminating the activity is achieved by inserting one or two nucleotides into the part of the coding sequence of said polypeptide corresponding to amino acids of positions 1 to 258 of the encoded amino acid sequence.

15. The bacterium as claimed in any of claims 1 to 8, wherein said eliminating the activity is achieved by inserting a gene coding for a polypeptide of the biosynthetic pathway of an L-amino acid selected from L-lysine, L-valine, L-threonine, L-isoleucine, L-histidine, L-proline and L-ornithine into the part of the coding sequence corresponding to amino acids of positions 175 to 258 of said polypeptide.

16. The bacterium of claim 15, wherein said L-amino acid is L-Lysine.

17. The bacterium as claimed in claim 15 or 16, wherein said gene coding for a polypeptide of the L-lysine biosynthetic pathway is selected from gene *pyc*, gene *aspB,* gene *lysC*, gene *asd,* gene *dapA*, gene *dapB*, gene *ddh*, gene *lysE*, gene *dapF* and gene *lysA.*

18. The bacterium as claimed in any of claims 1 to 8, wherein said eliminating the activity is achieved by substituting one or more codons coding for an amino acid of said polypeptide with a TAA-, TGA- or TAG- stop codon in the part of coding sequence corresponding to amino acids of positions 1 to 258 of the encoded amino acid sequence.

19. The bacterium as claimed in any of claims 1 to 8, wherein said eliminating the activity is achieved by inserting at least one stop codon selected from taa-, tga- or tag- stop codon between at least one pair of adjacent codons in the part of coding sequence corresponding to amino acids of positions 1 to 258 of the encoded amino acid sequence of said polypeptide.

20. A method for the fermentative production of an L-amino acid comprising the steps of
a) cultivating the bacterium as defined in any of claims 1 to 19 in a suitable medium under suitable conditions, and
b) accumulating said L-amino acid in the medium to form an L-amino acid containing fermentation broth.

21. The method of claim 20, wherein said method is selected from the group consisting of batch process, fed batch process and continuous process.

22. The method as claimed in any of claims 20 to 21, wherein said method comprises manufacturing an L-amino acid containing product from said fermentation broth.

23. The method as claimed in any of claims 20 to 22, wherein said method comprises extracting or substantially eliminating water from said fermentation broth.

24. The method of claim 23, wherein at least 40 % (w/w), preferred at least 90 % (w/w), more preferred at least 95 % (w/w) water are extracted from the fermentation broth.

25. The method of claim 22, wherein said manufacturing comprises a purification step.

26. The method of claim 25, wherein said purification step is selected from the group consisting of treatment with char coal, ionic exchange and crystallization.


SEQUENCE LISTING

<110>  Evonik Degussa GmbH

<120>  Process For The Fermentative Production of L-Amino Acids

<130>  file number: 201700286

<160>  16

<170>  PatentIn version 3.5

<210>  1
<211>  481
<212>  PRT
<213>  Corynebacterium glutamicum AS019E12

<220>
<221>  MISC_FEATURE
<222>  (1)..(481)
<223>  amino acid sequence disclosed in GenBank accession number
       AF237667

<400>  1

Met Asp Ser Gln Ile Asn Thr Gln Thr Ser Pro Ala Ala Ala Lys Leu
1               5                   10                  15

Pro Arg Glu Val Val Val Val Leu Ser Ile Leu Val Val Ser Ala Met
            20                  25                  30

Ile Met Ile Leu Asn Glu Thr Ile Leu Ser Val Ala Leu Pro Ser Ile
        35                  40                  45

Met Glu Asp Phe Ser Val Pro Glu Thr Thr Ala Gln Trp Leu Thr Thr
    50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Val Gln Ala Val Gly Thr Ala Leu Val Met
        115                 120                 125

Pro Leu Leu Met Thr Val Thr Leu Thr Val Val Pro Ala Glu Arg Arg
    130                 135                 140

Gly Ser Met Met Gly Ile Ile Ser Ile Val Ile Ser Val Ala Pro Ala

145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Val Ile Ala Leu Val Ile
            180                 185                 190

Gly Phe Phe Leu Ile Lys Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Glu Gly Thr Val
225                 230                 235                 240

Gly Ile Phe Ala Ile Val Val Gly Ala Ile Ala Leu Leu Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Ile Ala Ala Ser Ser Met Thr Phe Leu Asn Glu Asn Ser
        355                 360                 365

Pro Val Trp Met Val Val Val Met His Val Val Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Lys Val Ala Phe Ile Ala Gly Ala Ile Ile Ala Val
    450                 455                 460

Ile Ala Leu Val Val Ser Leu Phe Val Thr Arg Val Glu Glu Glu Ala
465                 470                 475                 480

His

<210>  2
<211>  481
<212>  PRT
<213>  Corynebacterium glutamicum ATCC13032

<220>
<221>  MISC_FEATURE
<222>  (1)..(481)
<223>  amino acid sequence disclosed in GenBank accession number
       BAC00079

<400>  2
   Met Asp Ser Gln Ile Asn Thr Gln Thr Ser Pro Ala Ala Ala Lys Leu
   1 5 10 15
   Pro Arg Glu Val Val Val Val Leu Ser Ile Leu Val Val Ser Ala Met
   20 25 30
   Ile Met Ile Leu Asn Glu Thr Ile Leu Ser Val Ala Leu Pro Ser Ile
   35 40 45
   Met Glu Asp Phe Ser Val Pro Glu Thr Thr Ala Gln Trp Leu Thr Thr
   50 55 60
   Gly Phe Met Leu Thr Met Ala Val Val Ile Pro Thr Thr Gly Tyr Leu
   65 70 75 80
   Leu Asp Arg Phe Ser Thr Lys Thr Ile Phe Val Thr Ala Leu Leu Phe
   85 90 95
   Phe Thr Val Gly Thr Leu Thr Ala Ala Leu Ala Pro Thr Phe Ala Val
   100 105 110
   Leu Leu Gly Ala Arg Ile Val Gln Ala Val Gly Thr Ala Leu Val Met
   115 120 125
   Pro Leu Leu Met Thr Val Thr Leu Thr Val Val Pro Ala Glu Arg Arg
   130 135 140
   Gly Ser Met Met Gly Ile Ile Ser Ile Val Ile Ser Val Ala Pro Ala
   145 150 155 160
   Leu Gly Pro Thr Leu Ser Gly Val Ile Leu Asn Ser Leu Thr Trp His
   165 170 175
   Trp Leu Phe Trp Met Met Leu Pro Ile Val Val Ile Ala Leu Val Ile
   180 185 190
   Gly Phe Phe Leu Ile Lys Asn Ile Gly Glu Thr Lys Ile Thr Pro Leu
   195 200 205
   Asp Val Leu Ser Val Ile Leu Ser Val Phe Ala Phe Gly Gly Leu Val
   210 215 220
   Tyr Gly Phe Ser Ser Phe Gly Ala Ile Leu Glu Gly Glu Gly Thr Val
   225 230 235 240
   Gly Ile Phe Ala Ile Val Val Gly Ala Ile Ala Leu Leu Ile Phe Ala
   245 250 255
   Leu Arg Gln His Gln Leu Gly Lys Gln Asp Lys Ala Leu Met Asp Leu
   260 265 270
   Arg Ala Phe Lys Val Arg Asn Phe Ser Phe Ser Leu Thr Thr Ile Leu
   275 280 285
   Leu Ala Phe Gly Ala Met Leu Gly Thr Val Met Val Leu Pro Ile Tyr
   290 295 300
   Leu Gln Thr Ser Leu Gly Val Thr Ala Leu Val Thr Gly Leu Val Val
   305 310 315 320
   Met Pro Gly Gly Leu Leu Gln Gly Leu Ile Ser Pro Phe Ile Gly Arg
   325 330 335
   Phe Tyr Asp Lys Val Gly Pro Arg Pro Leu Leu Ile Pro Gly Ala Ile
   340 345 350
   Ala Leu Ala Ile Ala Ala Ser Ser Met Thr Phe Leu Asn Glu Asn Ser
           355                 360                 365
   Pro Val Trp Met Val Val Val Met His Val Val Phe Ser Ile Gly Met
       370                 375                 380
   Cys Leu Met Met Thr Pro Leu Met Thr Thr Ala Leu Gly Ala Leu Pro
   385                 390                 395                 400
   Lys His Leu Tyr Gly His Gly Ser Ala Ile Leu Asn Thr Phe Gln Gln
                   405                 410                 415
   Leu Ala Gly Ala Ala Gly Thr Ala Ile Met Ile Ala Ala Leu Ser Phe
               420                 425                 430
   Gly Thr Ser Ile Ala Ala Ser Ser Gly Ser Ala His Ala Glu Ala Val
           435                 440                 445
   Ala Ala Gly Thr Lys Val Ala Phe Ile Ala Gly Ala Ile Ile Ala Val
       450                 455                 460
   Ile Ala Leu Val Val Ser Leu Phe Val Thr Arg Val Glu Glu Glu Ala
   465                 470                 475                 480
   His

<210>  3
<211>  481
<212>  PRT
<213>  Corynebacterium glutamicum AJ1511

<220>
<221>  MISC_FEATURE
<222>  (1)..(481)
<223>  amino acid sequence disclosed in GenBank accession number
       BAV24312

<400>  3

Met Asp Ser Gln Ile Asn Thr Gln Thr Ser Pro Ala Ala Ala Lys Leu
1               5                   10                  15

Pro Arg Glu Val Val Val Val Leu Ser Ile Leu Val Val Ser Ala Met
            20                  25                  30

Ile Met Ile Leu Asn Glu Thr Ile Leu Ser Val Ala Leu Pro Ser Ile
        35                  40                  45

Met Glu Asp Phe Ser Val Pro Glu Thr Thr Ala Gln Trp Leu Thr Thr

50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Val Gln Ala Val Gly Thr Ala Leu Val Met
        115                 120                 125

Pro Leu Leu Met Thr Val Thr Leu Thr Val Val Pro Ala Glu Arg Arg
    130                 135                 140

Gly Ser Met Met Gly Ile Ile Ser Ile Val Ile Ser Val Ala Pro Ala
145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Val Ile Ala Leu Val Ile
            180                 185                 190

Gly Tyr Phe Leu Ile Gln Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Glu Gly Thr Val
225                 230                 235                 240

Gly Ile Leu Ala Ile Val Val Gly Ala Ile Ala Leu Leu Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Ile Ala Ala Ser Ser Met Thr Phe Leu Asn Glu Asn Ser
        355                 360                 365

Pro Val Trp Met Val Val Val Met His Val Val Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Lys Val Ala Phe Ile Ala Gly Ala Ile Ile Ala Val
    450                 455                 460

Ile Ala Leu Val Val Ser Leu Phe Val Thr Arg Val Glu Glu Glu Thr
465                 470                 475                 480

His

<210>  4
<211>  481
<212>  PRT
<213>  Corynebacterium glutamicum ATCC13869

<220>
<221>  MISC_FEATURE
<222>  (1)..(481)
<223>  amino acid sequence disclosed in GenBank accession number
       ANU34602

<400>  4

Met Asp Ser Gln Ile Asn Thr Gln Thr Ser Pro Ala Ala Ala Lys Leu

1               5                   10                  15

Pro Arg Glu Val Val Val Val Leu Ser Ile Leu Val Val Ser Ala Met
            20                  25                  30

Ile Met Ile Leu Asn Glu Thr Ile Leu Ser Val Ala Leu Pro Ser Ile
        35                  40                  45

Met Glu Asp Phe Ser Val Pro Glu Thr Thr Ala Gln Trp Leu Thr Thr
    50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Val Gln Ala Val Gly Thr Ala Leu Val Met
        115                 120                 125

Pro Leu Leu Met Thr Val Thr Leu Thr Val Val Pro Ala Glu Arg Arg
    130                 135                 140

Gly Ser Met Met Gly Ile Ile Ser Ile Val Ile Ser Val Ala Pro Ala
145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Val Ile Ala Leu Val Ile
            180                 185                 190

Gly Tyr Phe Leu Ile Gln Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Glu Gly Thr Val
225                 230                 235                 240

Gly Ile Leu Ala Ile Val Val Gly Ala Ile Ala Leu Leu Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Ile Ala Ala Ser Ser Met Thr Phe Leu Asn Glu Asn Ser
        355                 360                 365

Pro Val Trp Met Val Val Val Met His Val Val Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Lys Val Ala Phe Ile Ala Gly Ala Ile Ile Ala Val
    450                 455                 460

Ile Ala Leu Val Val Ser Leu Phe Val Thr Arg Val Glu Glu Glu Thr
465                 470                 475                 480

His

<210> 5
<211> 481
<212> PRT

<213>  Corynebacterium glutamicum R

<220>
<221>  MISC_FEATURE
<222>  (1)..(481)
<223>  amino acid sequence disclosed in GenBank accession number
       BAF55600

<400>  5

Met Asp Ser Gln Ile Asn Thr Gln Thr Ser Pro Ala Thr Ala Lys Leu
1               5                   10                  15

Pro Arg Glu Val Val Val Val Leu Ser Ile Leu Val Ile Ser Ala Met
            20                  25                  30

Ile Met Ile Leu Asn Glu Thr Ile Leu Ser Val Ala Leu Pro Ser Ile
        35                  40                  45

Met Ala Asp Phe Gln Val Pro Glu Thr Thr Ala Gln Trp Leu Thr Thr
    50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Ile Gln Ala Val Gly Thr Ala Leu Val Met
        115                 120                 125

Pro Leu Leu Met Thr Val Thr Leu Thr Val Val Pro Ala Glu Arg Arg
    130                 135                 140

Gly Ser Met Met Gly Ile Ile Ser Ile Val Ile Ser Val Ala Pro Ala
145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Ala Val Ala Leu Val Ile
            180                 185                 190

Gly Phe Phe Leu Ile Lys Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Lys Gly Val Val
225                 230                 235                 240

Gly Ile Val Ala Ile Val Val Gly Ala Ile Ala Leu Val Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Val Ala Ala Ser Ser Met Thr Phe Leu Asn Glu Asn Ser
        355                 360                 365

Pro Val Trp Met Val Val Val Leu His Val Ile Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Arg Val Ala Phe Ile Ala Gly Ala Ile Ile Ala Val
    450                 455                 460

Ile Ala Leu Val Val Ser Leu Phe Val Thr Arg Val Glu Glu Glu Thr
465                 470                 475                 480

His

<210>  6
<211>  481
<212>  PRT
<213>  Corynebacterium glutamicum ATCC14067

<220>
<221>  MISC_FEATURE
<222>  (1)..(481)
<223>  amino acid sequence disclosed in GenBank accession number
       KEI24239

<400>  6

Met Asp Ser Gln Ile Asn Thr Gln Thr Ser Pro Ala Ala Ala Lys Leu
1               5                   10                  15

Pro Arg Glu Val Val Val Val Leu Ser Ile Leu Val Ile Ser Ala Met
            20                  25                  30

Ile Met Ile Leu Asn Glu Thr Ile Leu Ser Val Ala Leu Pro Ser Ile
        35                  40                  45

Met Ala Asp Phe Gln Val Pro Glu Thr Thr Ala Gln Trp Leu Thr Thr
    50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Ile Gln Ala Val Gly Thr Ala Leu Val Met
        115                 120                 125

Pro Leu Leu Met Thr Val Thr Leu Thr Val Val Pro Ala Glu Arg Arg
    130                 135                 140

Ser Ser Met Met Gly Ile Ile Ser Ile Val Ile Ser Val Ala Pro Ala
145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Ala Val Ala Leu Val Ile
            180                 185                 190

Gly Phe Phe Leu Ile Lys Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Glu Gly Thr Val
225                 230                 235                 240

Gly Ile Leu Ala Ile Val Val Gly Ala Ile Ala Leu Leu Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Ile Ala Ala Ser Ser Met Thr Phe Leu Asn Glu Asn Ser
        355                 360                 365

Pro Val Trp Met Val Val Val Met His Val Val Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Lys Val Ala Phe Ile Ala Gly Ala Ile Ile Ala Val
    450                 455                 460

Ile Ala Leu Val Ile Ser Leu Phe Val Thr Arg Val Glu Glu Glu Thr
465                 470                 475                 480

His

<210>  7
<211>  3446
<212>  DNA
<213>  Corynebacterium glutamicum ATCC13032

<220>
<221>  misc_feature
<222>  (1)..(3446)
<223>  nucleotide sequence comprising locus_tag NCgl2592 disclosed in
        GenBank accession number NC_003450

<220>
<221>  misc_feature
<222>  (1)..(1000)
<223>  nucleotide sequence upstream of cds

<220>
<221>  misc_feature
<222>  (201)..(201)
<223>  nucleobase adenine

<220>
<221>  misc_feature
<222>  (988)..(988)
<223>  nucleobase cytosine

<220>
<221>  CDS
<222>  (1001)..(2443)

<220>
<221>  misc_feature
<222>  (1311)..(1311)
<223>  nucleobase cytosine

<220>
<221>  misc_feature
<222>  (2444)..(2446)
<223>  stop codon

<220>

<221>  misc_feature
<222>  (2444)..(3446)
<223>  nucleotide downstream of cds

<220>
<221>  misc_feature
<222>  (2456)..(2456)
<223>  nucleobase adenine

<220>
<221>  misc_feature
<222>  (3246)..(3246)
<223>  nucleobase thymine

<400>  7
caaatgatta aactcactca tgaagtcgca atcaatgctg aaaagtaccg aagccttcct      60

gcatgggata aagcagccgc ccgagctttt gcagtgggta tgactgtgga caaagcagtg     120

gtagcggggc agtctgcggc gaggttatgg ggataccaaa ctttgactgt tgagaaaacc     180

gtgttgtgtc tgttgccgga aaggctacgt tcaaaatcct ccaagcattg gccgtccggg     240

atgcgatata aagatcgtta cctctcgtcg cgtgatattc gagaggttca tgggatccga     300

gttacgggag cgttccgcac atttttggac atcgctttgg atgatggggt ggtggcggct     360

gtggtcacta ttgattcagc tcgaagacag aatccatcgc ttacgcgtga gaagttaatg     420

cacagtgcgg aaagtttccc gaggcatcgg ggtgtgaagg cgtatcggca ggcgattgag     480

ttgtcgattc ccaattcgga tagtgctcag gagacgaggg ctcggttaat ccttcgggag     540

gccaagctcc cggaaatcca gtcagtgaag gtgcaggccc gtttcgatca atcgcacaac     600

aagtatttcc tcgtcgattt cttgatcaat gagtggatca tcgtggagat tgatggacgt     660

tcgaaatatg attccccgga gctcaatgag gtgctcatgg ctgaacgcga tcgggagaaa     720

ttcttcctca atcagggcta tgcggtctta agaatcgatc cgaaacagtt agacctcaac     780

caagatgggg agtgtgagtt catcggaatc ctcaaaaaca ctttgcagaa gaccccacct     840

gagcacctca agcaagccgc ctaaacactc caagaaccac catttgcaca atgcacttgc     900

ttgtggcact ctttagtagt tttttctcat agctcagttt cgcaacttta gagaactcta     960

gaaactgagc ttcatgctgt gaaaggcctt ttctccattc atg gat tcc caa att      1015
                                            Met Asp Ser Gln Ile
                                            1               5

aat act cag acc tct ccg gca gct gcg aag ctg cct agg gag gtc gtt      1063
Asn Thr Gln Thr Ser Pro Ala Ala Ala Lys Leu Pro Arg Glu Val Val
                10                  15                  20

gtt gtt ctt tcg atc ctc gtg gtt tcc gcg atg atc atg att ctt aat      1111
Val Val Leu Ser Ile Leu Val Val Ser Ala Met Ile Met Ile Leu Asn
            25                  30                  35

gaa acc att ctg tcg gtt gcg ttg cct tcc atc atg gaa gat ttc tcc      1159
Glu Thr Ile Leu Ser Val Ala Leu Pro Ser Ile Met Glu Asp Phe Ser
        40                  45                  50

gtg cct gaa act act gca cag tgg ttg acc act ggc ttt atg ttg acg          1207
Val Pro Glu Thr Thr Ala Gln Trp Leu Thr Thr Gly Phe Met Leu Thr
    55                  60                  65

atg gca gtg gtg att cca act act ggt tat ctg ctt gat cgt ttt tcc          1255
Met Ala Val Val Ile Pro Thr Thr Gly Tyr Leu Leu Asp Arg Phe Ser
70                  75                  80                  85

act aag acg atc ttt gtt act gcg ttg ttg ttc ttt acg gtt ggt acg          1303
Thr Lys Thr Ile Phe Val Thr Ala Leu Leu Phe Phe Thr Val Gly Thr
                90                  95                  100

ttg act gcg gcg ttg gct cca acg ttt gcg gtg ctg ctt ggt gct cgt          1351
Leu Thr Ala Ala Leu Ala Pro Thr Phe Ala Val Leu Leu Gly Ala Arg
            105                 110                 115

atc gtt cag gcg gtt ggt act gcg ctg gtg atg cct ttg ctg atg acg          1399
Ile Val Gln Ala Val Gly Thr Ala Leu Val Met Pro Leu Leu Met Thr
        120                 125                 130

gtt acg ttg acg gtg gtt cct gcg gag cgt cgt ggt tcg atg atg ggc          1447
Val Thr Leu Thr Val Val Pro Ala Glu Arg Arg Gly Ser Met Met Gly
    135                 140                 145

att att tcc atc gtg att tct gtt gcg ccg gcg ctt ggt cct acg ttg          1495
Ile Ile Ser Ile Val Ile Ser Val Ala Pro Ala Leu Gly Pro Thr Leu
150                 155                 160                 165

tct ggt gtc att ctt aac tct ttg acc tgg cac tgg ttg ttt tgg atg          1543
Ser Gly Val Ile Leu Asn Ser Leu Thr Trp His Trp Leu Phe Trp Met
                170                 175                 180

atg ctt ccg atc gtt gtt atc gct ttg gta att ggt ttc ttc ttg atc          1591
Met Leu Pro Ile Val Val Ile Ala Leu Val Ile Gly Phe Phe Leu Ile
            185                 190                 195

aaa aat atc ggc gaa acc aag atc acc cca ctg gat gtt ctg tct gtc          1639
Lys Asn Ile Gly Glu Thr Lys Ile Thr Pro Leu Asp Val Leu Ser Val
        200                 205                 210

atc ctt tcg gtg ttt gcc ttc ggt ggt ttg gtg tac ggc ttc agt tcc          1687
Ile Leu Ser Val Phe Ala Phe Gly Gly Leu Val Tyr Gly Phe Ser Ser
    215                 220                 225

ttc gga gca atc ctg gag ggc gaa ggc acc gta ggt atc ttc gcg atc          1735
Phe Gly Ala Ile Leu Glu Gly Glu Gly Thr Val Gly Ile Phe Ala Ile
230                 235                 240                 245

gtc gtt ggc gcc atc gca ctc ctc atc ttt gct ttg cga cag cac caa          1783
Val Val Gly Ala Ile Ala Leu Leu Ile Phe Ala Leu Arg Gln His Gln
                250                 255                 260

ctc ggc aag caa gac aaa gca ctg atg gat ctc cga gcc ttc aag gtg          1831
Leu Gly Lys Gln Asp Lys Ala Leu Met Asp Leu Arg Ala Phe Lys Val
            265                 270                 275

agg aac ttc agc ttc tcc ttg acc acc atc ctt ttg gcg ttc ggc gcg          1879
Arg Asn Phe Ser Phe Ser Leu Thr Thr Ile Leu Leu Ala Phe Gly Ala
        280                 285                 290

atg ctc gga acc gtc atg gtt ttg cca atc tac ctg cag act tcc ctc          1927
Met Leu Gly Thr Val Met Val Leu Pro Ile Tyr Leu Gln Thr Ser Leu
    295                 300                 305

gga gtt act gct ttg gtg acc ggt ttg gtt gtt atg ccc ggc ggc ctc 1975
Gly Val Thr Ala Leu Val Thr Gly Leu Val Val Met Pro Gly Gly Leu
310                 315                 320                 325

ctc cag ggt ctg atc agc cca ttc atc gga cgt ttc tac gac aag gtc 2023
Leu Gln Gly Leu Ile Ser Pro Phe Ile Gly Arg Phe Tyr Asp Lys Val
                330                 335                 340

ggt cca cgt ccg ctg ctg att ccc gga gca att gcg ctg gct atc gcg 2071
Gly Pro Arg Pro Leu Leu Ile Pro Gly Ala Ile Ala Leu Ala Ile Ala
            345                 350                 355

gca tcc tcg atg act ttc ctc aat gag aat tca ccc gtg tgg atg gtc 2119
Ala Ser Ser Met Thr Phe Leu Asn Glu Asn Ser Pro Val Trp Met Val
        360                 365                 370

gtg gtc atg cac gtt gtg ttc agc atc ggc atg tgt ttg atg atg acc 2167
Val Val Met His Val Val Phe Ser Ile Gly Met Cys Leu Met Met Thr
    375                 380                 385

cct ctc atg acc acc gct ctc ggc gcc ctt ccg aag cac ctc tat ggt 2215
Pro Leu Met Thr Thr Ala Leu Gly Ala Leu Pro Lys His Leu Tyr Gly
390                 395                 400                 405

cac ggc tcc gca att ttg aac acg ttc caa cag ctc gca ggc gca gcc 2263
His Gly Ser Ala Ile Leu Asn Thr Phe Gln Gln Leu Ala Gly Ala Ala
                410                 415                 420

gga aca gcg atc atg att gca gca ctt tcc ttc ggc act tcc att gca 2311
Gly Thr Ala Ile Met Ile Ala Ala Leu Ser Phe Gly Thr Ser Ile Ala
            425                 430                 435

gcg tct tcg gga tct gcg cat gct gaa gct gtt gcc gct ggt acc aag 2359
Ala Ser Ser Gly Ser Ala His Ala Glu Ala Val Ala Ala Gly Thr Lys
        440                 445                 450

gtt gcg ttc atc gca ggc gca atc atc gcg gtg atc gct ttg gtt gtt 2407
Val Ala Phe Ile Ala Gly Ala Ile Ile Ala Val Ile Ala Leu Val Val
    455                 460                 465

tcc ctc ttc gtc act cgc gtc gag gaa gaa gct cac taaataccaa 2453
Ser Leu Phe Val Thr Arg Val Glu Glu Glu Ala His
470                 475                 480

aaaatggggc agaaggtcat tgactttctg ccccattttt ttgcttctcg acgcctcccg 2513

ccgccacctc accttaagcg tgctcagcgc tccaggttct gttctcgcca cccggtttgt 2573

ggttcccaca ccaacagctt gttggtaggt cggctttctg ggtttttcag tgaaataaca 2633

attttggttc gggaagaata tgccactgaa aaactattcc tgtctaagtt cacagttgtt 2693

atttgttctg ttgtttcagg gtcgtggaaa gcatatgtgc cttcttgctc atgaaacatc 2753

atgagtgttc cggtgggaaa accgagtgtt ggagaatccc caggtggaag ataaattaga 2813

ccagattcgg gaacctcagg gtcgccaaag acttctacgt tgagaatctc gaggtcctgg 2873

ttaaacgtga tgtggtggtt actagcgcgt aagtaaattc gatcaccgta tgggtaaatt 2933

gctgtgatta tcgatcggcc gatatcaaga tctactgctt taaatggttt aagcctaagg 2993

attgtttgaa gccaaaagtt gtcattaaga cgagggtttt cgcgtgtgac attgtgcaag 3053

tttttcccga agctgtctaa caatctccaa cctgctggaa tcgaagggac agcttcgaat 3113

acttctttaa gatccggaag ctcccatgtt tttcccttgc aatcactgaa atcgtgaggc 3173

cattgctttt gttttacgcg ggctctatta gcggaatcga ggacgatctc ttggaacgtg 3233

gggatggaga tctcgtgtaa tagttcgcct gttgacacgc tgaatccacg gacgaaaggc 3293

aagctttcat cggagagcca aaatatgtca ccgtcactgg tactagtgct gatgccttca 3353

agagtttctt ggtttttggg tggatgcaca ccatctaggg tcacgtcgat aattgcgtcc 3413

gtaccgtggt agtgcgctgc tgtaacgact gca 3446

<210> 8
<211> 481
<212> PRT
<213> Corynebacterium glutamicum ATCC13032

<400> 8

Met Asp Ser Gln Ile Asn Thr Gln Thr Ser Pro Ala Ala Ala Lys Leu
1               5                   10                  15

Pro Arg Glu Val Val Val Val Leu Ser Ile Leu Val Val Ser Ala Met
            20                  25                  30

Ile Met Ile Leu Asn Glu Thr Ile Leu Ser Val Ala Leu Pro Ser Ile
        35                  40                  45

Met Glu Asp Phe Ser Val Pro Glu Thr Thr Ala Gln Trp Leu Thr Thr
    50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Val Gln Ala Val Gly Thr Ala Leu Val Met
        115                 120                 125

Pro Leu Leu Met Thr Val Thr Leu Thr Val Val Pro Ala Glu Arg Arg
    130                 135                 140

Gly Ser Met Met Gly Ile Ile Ser Ile Val Ile Ser Val Ala Pro Ala
145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Val Ile Ala Leu Val Ile
            180                 185                 190

Gly Phe Phe Leu Ile Lys Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Glu Gly Thr Val
225                 230                 235                 240

Gly Ile Phe Ala Ile Val Val Gly Ala Ile Ala Leu Leu Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Ile Ala Ala Ser Ser Met Thr Phe Leu Asn Glu Asn Ser
        355                 360                 365

Pro Val Trp Met Val Val Val Met His Val Val Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Lys Val Ala Phe Ile Ala Gly Ala Ile Ile Ala Val
    450                 455                 460

Ile Ala Leu Val Val Ser Leu Phe Val Thr Arg Val Glu Glu Glu Ala
465                 470                 475                 480

His

<210>  9
<211>  3446
<212>  DNA
<213>  Corynebacterium glutamicum ATCC13869

<220>
<221>  misc_feature
<222>  (1)..(3446)
<223>  nucleotide sequence comprising locus_tag BBD29_13115 disclosed in
        GenBank accession number NZ_CP016335

<220>
<221>  misc_feature
<222>  (1)..(1000)
<223>  nucleotide sequence upstream of cds

<220>
<221>  CDS
<222>  (1001)..(2443)

<220>
<221>  misc_feature
<222>  (2444)..(2446)
<223>  stop codon

<220>
<221>  misc_feature
<222>  (2444)..(3446)
<223>  nucleotide sequence downstream of cds

<400>  9
caaatgatta aactcactca tgaagtcgca atcaatgctg aaaagtaccg aagccttcct      60

gcatgggata aagcagccgc ccgagctttt gcagtgggta tgactgtgga caaagcagtg     120

gtagcggggc agtctgcggc gaggttatgg ggataccaaa ctttgactgt tgagaaaacc     180

gtgttgtgtc tgttgccgga aaggctacgt tcaaaatcct ccaagcattg gccgtccggg     240

atgcgatata aagatcgtta cctctcgccg cgtgatattc gggaagttca tgggattcga     300

gtcacgggag ctttccgcac atttttggac atcgctttgg atgatggggt ggtggcggct 360

gtggtcacta ttgattcagc tcgaaggcag aatccgtcgc ttacgcgtga gaagttgatg 420

tgcagtgcag aaagtttccc gaggcatcgg ggtgtgaagg cgtatcggca ggcgattgag 480

ttgtcgattc ccaattcgga tagtgctcag gagacgaggg ctcggttgat cctgggggag 540

gccaacctcc cggagatcca gtcagtgcag gtgcaagccc gtttcgatca atcgcacaac 600

aagtatttcc tcgtggattt cttaatcaat gagtggatca tcgtggagat tgatggacgt 660

tcgaaatatg attctccaga gcttaatgag gtgctcatgg ctgaacgtga tcgggagaaa 720

ttcttcctca accaaggata tgcagtgctg agaattgatc caaaacaatt agaccccgac 780

caaaacggcg agtgtgaatt catcggaatc ctcaaaaaca ctttgcagaa gaccccgcca 840

gagcacctca agcaagccgc ctaaacactc caagaaccac catttgcaca atgcacttgc 900

ttgtggcact ctttagtagt tttttctcat agctcagttt cgcaacttta gagaactcta 960

gaaactgagc ttcatgctgt gaaaggcctt ttctccattc atg gat tcc caa att 1015
Met Asp Ser Gln Ile
1               5

aat act cag acc tct ccg gca gct gcg aag ctg cct agg gag gtc gtt 1063
Asn Thr Gln Thr Ser Pro Ala Ala Ala Lys Leu Pro Arg Glu Val Val
                10                  15                  20

gtt gtt ctt tcg atc ctc gtg gtt tcc gcg atg atc atg att ctt aat 1111
Val Val Leu Ser Ile Leu Val Val Ser Ala Met Ile Met Ile Leu Asn
            25                  30                  35

gaa acc att ctg tcg gtt gcg ttg cct tcc atc atg gaa gat ttc tcc 1159
Glu Thr Ile Leu Ser Val Ala Leu Pro Ser Ile Met Glu Asp Phe Ser
        40                  45                  50

gtg cct gaa act act gca cag tgg ttg acc act ggc ttt atg ttg acg 1207
Val Pro Glu Thr Thr Ala Gln Trp Leu Thr Thr Gly Phe Met Leu Thr
    55                  60                  65

atg gca gtg gtg att cct act act ggt tat ctg ctt gat cgt ttt tcc 1255
Met Ala Val Val Ile Pro Thr Thr Gly Tyr Leu Leu Asp Arg Phe Ser
70                  75                  80                  85

act aag acg atc ttt gtt act gcg ttg ttg ttc ttt acg gtt ggt acg 1303
Thr Lys Thr Ile Phe Val Thr Ala Leu Leu Phe Phe Thr Val Gly Thr
                90                  95                  100

ttg act gcg gcg ttg gcc cca acg ttt gct gtg ctg ctt ggt gct cgt 1351
Leu Thr Ala Ala Leu Ala Pro Thr Phe Ala Val Leu Leu Gly Ala Arg
            105                 110                 115

atc gtt cag gcg gtt ggt act gcg ctg gtg atg cct ttg ctg atg aca 1399
Ile Val Gln Ala Val Gly Thr Ala Leu Val Met Pro Leu Leu Met Thr
        120                 125                 130

gtt acg ttg acg gtg gtt cct gcg gag cgt cgt ggt tcg atg atg ggc 1447
Val Thr Leu Thr Val Val Pro Ala Glu Arg Arg Gly Ser Met Met Gly
    135                 140                 145

att att tcc atc gtg att tct gtt gcg ccg gcg ctt ggt cct act ttg 1495
Ile Ile Ser Ile Val Ile Ser Val Ala Pro Ala Leu Gly Pro Thr Leu
150                 155                 160                 165

tct ggt gtc att ctt aac tct ttg acc tgg cac tgg ttg ttt tgg atg 1543
Ser Gly Val Ile Leu Asn Ser Leu Thr Trp His Trp Leu Phe Trp Met
                170                 175                 180

atg ctt ccg atc gtt gtt atc gct ttg gtg att ggc tac ttc ctg atc 1591
Met Leu Pro Ile Val Val Ile Ala Leu Val Ile Gly Tyr Phe Leu Ile
            185                 190                 195

caa aac att ggc gaa acc aag atc act cca ctg gat gtt ctg tct gtc 1639
Gln Asn Ile Gly Glu Thr Lys Ile Thr Pro Leu Asp Val Leu Ser Val
        200                 205                 210

atc ctt tcg gtg ttt gcc ttc ggt ggt ttg gtg tac ggc ttc agt tcc 1687
Ile Leu Ser Val Phe Ala Phe Gly Gly Leu Val Tyr Gly Phe Ser Ser
    215                 220                 225

ttc gga gca atc ctg gag ggc gaa ggt acc gta ggt atc ctc gcg atc 1735
Phe Gly Ala Ile Leu Glu Gly Glu Gly Thr Val Gly Ile Leu Ala Ile
230                 235                 240                 245

gtc gtt ggc gcc atc gca ctt ctc atc ttt gct ttg cga cag cac caa 1783
Val Val Gly Ala Ile Ala Leu Leu Ile Phe Ala Leu Arg Gln His Gln
                250                 255                 260

ctt ggc aaa caa gac aaa gcc ctc atg gat ttg cgt gct ttc aag gtg 1831
Leu Gly Lys Gln Asp Lys Ala Leu Met Asp Leu Arg Ala Phe Lys Val
            265                 270                 275

agg aac ttc agt ttc tcc ttg acc acc atc ctt ttg gcg ttc ggt gcg 1879
Arg Asn Phe Ser Phe Ser Leu Thr Thr Ile Leu Leu Ala Phe Gly Ala
        280                 285                 290

atg ctc gga acc gtc atg gtt ttg ccc atc tac ctg cag act tcc ctc 1927
Met Leu Gly Thr Val Met Val Leu Pro Ile Tyr Leu Gln Thr Ser Leu
    295                 300                 305

gga gtt act gct ttg gtg acc ggt ttg gtt gtt atg ccc ggc ggc ctc 1975
Gly Val Thr Ala Leu Val Thr Gly Leu Val Val Met Pro Gly Gly Leu
310                 315                 320                 325

ctc cag ggt ctg atc agc cca ttc atc gga cgt ttc tac gac aag gtc 2023
Leu Gln Gly Leu Ile Ser Pro Phe Ile Gly Arg Phe Tyr Asp Lys Val
                330                 335                 340

ggt cca cgt ccg ctg ctg att ccc gga gca att gcg ctg gct atc gcg 2071
Gly Pro Arg Pro Leu Leu Ile Pro Gly Ala Ile Ala Leu Ala Ile Ala
            345                 350                 355

gca tcc tcg atg act ttc ctc aat gag aat tca cct gtg tgg atg gtc 2119
Ala Ser Ser Met Thr Phe Leu Asn Glu Asn Ser Pro Val Trp Met Val
        360                 365                 370

gtg gtc atg cac gtt gtg ttc agc atc ggt atg tgt ttg atg atg acc 2167
Val Val Met His Val Val Phe Ser Ile Gly Met Cys Leu Met Met Thr
    375                 380                 385

cct ctc atg acc acc gct ctc ggc gcc ctt ccg aag cac ctc tat ggt 2215
Pro Leu Met Thr Thr Ala Leu Gly Ala Leu Pro Lys His Leu Tyr Gly
390                 395                 400                 405

cac ggc tcc gca att ttg aac acg ttc caa cag ctc gca ggt gca gcc 2263
His Gly Ser Ala Ile Leu Asn Thr Phe Gln Gln Leu Ala Gly Ala Ala
                410                 415                 420

gga aca gcg atc atg att gca gca ctt tcc ttc ggc act tcc att gca 2311
Gly Thr Ala Ile Met Ile Ala Ala Leu Ser Phe Gly Thr Ser Ile Ala
            425                 430                 435

gcg tct tcc gga tct gcg cat gct gaa gct gtt gcc gct ggt acc aag 2359
Ala Ser Ser Gly Ser Ala His Ala Glu Ala Val Ala Ala Gly Thr Lys
        440                 445                 450

gtt gcg ttc atc gca ggc gca atc atc gcg gtg atc gct ttg gtt gtt 2407
Val Ala Phe Ile Ala Gly Ala Ile Ile Ala Val Ile Ala Leu Val Val
    455                 460                 465

tcc ctc ttc gtc act cgc gtt gag gaa gaa act cac taaacgctca 2453
Ser Leu Phe Val Thr Arg Val Glu Glu Glu Thr His
470                 475                 480

ataatggggc agacggtcat tgactttctg ccccggtttt ttgcttttcg acgcctccct 2513

gcagcgcacc ttgcaggtgc gcggggctta agcgtcgaaa gccctagatt tctacagaga 2573

catcgtcgaa tttaatgttg ctccattgac cgtctctaaa cactgagaca gaggaaggta 2633

ctggtttgag ataatcagta ccagtaggcg gggtaaccag tatgttgaag tggtttggtg 2693

acagaattct gacctcggtg aagtaccctt ccggtgttgt tcacctggtt atttcgcttc 2753

ctgaaatctg atcgagaaat acgatttcat cttttttcgt gaacttggca gctacgcctt 2813

cttgtgtaat ccagcgttca tgcttcgcac caagctgatg ttcttcggca ctttgcactt 2873

gaaagtttga atccaaaaag acggtcatgc tccagaattg cacaaagtac aggtcttcta 2933

ctcggcatgc gtttaggatc ctggatccgt cattgctgat tacacagatt tccaattgtc 2993

ctgctggagt gcttcgaccc agtgctgttt gctctccatc ggtcagggag taaagcccat 3053

cgagaaagct ttcttgtaat tcccaaggcg ctggaacgtc gagttctatg acaggctcta 3113

tccatggttg aggttccgca agcatgcgta ctctaggatt cagtgaccag gcgttttcat 3173

aatcgtgaat caacccttcc gaaaactgaa gcggagaccc gtgagaaacc gaaataggca 3233

cataggtttg ccccaggtat tttccggttt ggagatccca gcatcgaaga attggaagca 3293

tctgatcaag gacccatagt tgttcattga tgatgtgagt gatggatgct tcaaactgtt 3353

gtggctgaag tggtggtgga actgtgccgt caagggtggc atcgacgata acttccaaca 3413

gccagctgcc attttctgtg taggcatcgt ctg 3446

<210> 10
<211> 481
<212> PRT
<213> Corynebacterium glutamicum ATCC13869

<400> 10
   Met Asp Ser Gln Ile Asn Thr Gln Thr Ser Pro Ala Ala Ala Lys Leu
   1               5                   10                  15
   Pro Arg Glu Val Val Val Val Leu Ser Ile Leu Val Val Ser Ala Met
               20                  25                  30
   Ile Met Ile Leu Asn Glu Thr Ile Leu Ser Val Ala Leu Pro Ser Ile
           35                  40                  45
   Met Glu Asp Phe Ser Val Pro Glu Thr Thr Ala Gln Trp Leu Thr Thr
       50                  55                  60
   Gly Phe Met Leu Thr Met Ala Val Val Ile Pro Thr Thr Gly Tyr Leu
   65                  70                  75                  80
   Leu Asp Arg Phe Ser Thr Lys Thr Ile Phe Val Thr Ala Leu Leu Phe
                   85                  90                  95
   Phe Thr Val Gly Thr Leu Thr Ala Ala Leu Ala Pro Thr Phe Ala Val
               100                 105                 110
   Leu Leu Gly Ala Arg Ile Val Gln Ala Val Gly Thr Ala Leu Val Met
           115                 120                 125
   Pro Leu Leu Met Thr Val Thr Leu Thr Val Val Pro Ala Glu Arg Arg
       130                 135                 140
   Gly Ser Met Met Gly Ile Ile Ser Ile Val Ile Ser Val Ala Pro Ala
   145                 150                 155                 160
   Leu Gly Pro Thr Leu Ser Gly Val Ile Leu Asn Ser Leu Thr Trp His
                   165                 170                 175
   Trp Leu Phe Trp Met Met Leu Pro Ile Val Val Ile Ala Leu Val Ile
               180                 185                 190
   Gly Tyr Phe Leu Ile Gln Asn Ile Gly Glu Thr Lys Ile Thr Pro Leu
           195                 200                 205
   Asp Val Leu Ser Val Ile Leu Ser Val Phe Ala Phe Gly Gly Leu Val
       210                 215                 220
   Tyr Gly Phe Ser Ser Phe Gly Ala Ile Leu Glu Gly Glu Gly Thr Val
   225                 230                 235                 240
   Gly Ile Leu Ala Ile Val Val Gly Ala Ile Ala Leu Leu Ile Phe Ala
                   245                 250                 255
   Leu Arg Gln His Gln Leu Gly Lys Gln Asp Lys Ala Leu Met Asp Leu
               260                 265                 270
   Arg Ala Phe Lys Val Arg Asn Phe Ser Phe Ser Leu Thr Thr Ile Leu
           275                 280                 285
   Leu Ala Phe Gly Ala Met Leu Gly Thr Val Met Val Leu Pro Ile Tyr
       290                 295                 300
   Leu Gln Thr Ser Leu Gly Val Thr Ala Leu Val Thr Gly Leu Val Val
   305                 310                 315                 320
   Met Pro Gly Gly Leu Leu Gln Gly Leu Ile Ser Pro Phe Ile Gly Arg
                   325                 330                 335
   Phe Tyr Asp Lys Val Gly Pro Arg Pro Leu Leu Ile Pro Gly Ala Ile
               340                 345                 350
   Ala Leu Ala Ile Ala Ala Ser Ser Met Thr Phe Leu Asn Glu Asn Ser
           355                 360                 365
   Pro Val Trp Met Val Val Val Met His Val Val Phe Ser Ile Gly Met
       370                 375                 380
   Cys Leu Met Met Thr Pro Leu Met Thr Thr Ala Leu Gly Ala Leu Pro
   385                 390                 395                 400
   Lys His Leu Tyr Gly His Gly Ser Ala Ile Leu Asn Thr Phe Gln Gln
                   405                 410                 415
   Leu Ala Gly Ala Ala Gly Thr Ala Ile Met Ile Ala Ala Leu Ser Phe
               420                 425                 430
   Gly Thr Ser Ile Ala Ala Ser Ser Gly Ser Ala His Ala Glu Ala Val
           435                 440                 445
   Ala Ala Gly Thr Lys Val Ala Phe Ile Ala Gly Ala Ile Ile Ala Val
       450                 455                 460
   Ile Ala Leu Val Val Ser Leu Phe Val Thr Arg Val Glu Glu Glu Thr
   465                 470                 475                 480
   His

<210> 11
<211> 3446

<212>  DNA
<213>  Corynebacterium glutamicum ATCC14067

<220>
<221>  misc_feature
<222>  (1)..(3446)
<223>  nucleotide sequence comprising locus_tag KIQ_001365 disclosed in
       GenBank accession number AGQQ02000002

<220>
<221>  misc_feature
<222>  (1)..(1000)
<223>  nucleotide sequence upstream of cds

<220>
<221>  CDS
<222>  (1001)..(2443)

<220>
<221>  misc_feature
<222>  (2444)..(2446)
<223>  stop codon

<220>
<221>  misc_feature
<222>  (2444)..(3446)
<223>  nucleotide sequence downstream of cds

<400>  11
tacccttccc tcaacgaagc cctcgcacgc aaattcccag gacaaccaga agtaaccatc      60

gactccaccg tcgaagaact ccgcgaaatc gccaaggtaa tcggcctctc cgtccccgaa     120

aatggcggct ggggacacgg caaactcgtc gaagaaatct gggaactcct ctgcgaagac     180

caactctacg gaccaatctt tgtcaaagac ttcccagtag aaacctcccc actcacacgc     240

caacaccgca ccaagccagg cgtcaccgaa aagtgggacc tctacgtccg cggatttgaa     300

ctagcaaccg gatactccga actcatcgac ccagtcatcc aacgcgaacg cttcgaagac     360

caagcccgcc tcgccgccga cggagacgac gaagccatgg tcctcgacga agacttcctc     420

accgcaatgg aacaaggcat gccaccaacc tccggcaacg gcatgggaat cgaccgcctc     480

ctcatggccc tcaccggcct cggaatccgc gaaaccgtac tcttcccaat ggtgaaacca     540

gaacaaaagt aggttttctg tttgggtagg ttagctccag gactttgtgt cctggagttt     600

tcctcttcct agggttgatc gggatgggtt ttgtaaggtc gggtcggcgt cggtccgtaa     660

gttcgatttc acggtttcga gaccttaaca gcgatttcac gggtttcggt ttctcgcgtt     720

caatttcttg tactcaattt cgcatattcg ataccactag gaaagacgtc aaaatccgtt     780

agacctcgac caaaacggcg agtgcgagtt catcggaatc ctcaaaaaca ctttgcagaa     840

gaccccacca gagcacctca ggcaagccgc ctaaacactc caagaaccac cacttgcaca     900

atgcacttgc ttatggcact ctttaggagt tttttctcat agctcagttt cgtaacacta     960

gaaactgagc ttcatgctgt gaaaggcttt ttctccattc atg gat tcc caa att      1015

Met Asp Ser Gln Ile
1               5

aat act cag acc tct ccg gca gct gcg aag ttg cct agg gag gtc gtt     1063
Asn Thr Gln Thr Ser Pro Ala Ala Ala Lys Leu Pro Arg Glu Val Val
            10                  15                  20

gtt gtt ctt tcg atc ctc gtg att tct gcg atg atc atg att ctt aat     1111
Val Val Leu Ser Ile Leu Val Ile Ser Ala Met Ile Met Ile Leu Asn
        25                  30                  35

gaa act atc ctg tcg gtc gcg ttg ccg tcg atc atg gcg gat ttc cag     1159
Glu Thr Ile Leu Ser Val Ala Leu Pro Ser Ile Met Ala Asp Phe Gln
    40                  45                  50

gtt cca gaa act act gcg cag tgg ttg acc act ggc ttt atg ttg acg     1207
Val Pro Glu Thr Thr Ala Gln Trp Leu Thr Thr Gly Phe Met Leu Thr
    55                  60                  65

atg gca gtg gtg att cct act act ggt tat ttg ctt gat cgt ttt tcc     1255
Met Ala Val Val Ile Pro Thr Thr Gly Tyr Leu Leu Asp Arg Phe Ser
70                  75                  80                  85

act aag acg atc ttt gtt act gcg ttg ttg ttc ttt act gtt ggt acc     1303
Thr Lys Thr Ile Phe Val Thr Ala Leu Leu Phe Phe Thr Val Gly Thr
            90                  95                  100

ttg act gcg gcg ttg gcc cca acg ttt gct gtg ctg ctt ggt gct cgt     1351
Leu Thr Ala Ala Leu Ala Pro Thr Phe Ala Val Leu Leu Gly Ala Arg
        105                 110                 115

atc att cag gcg gtt ggt act gcg ctg gtg atg cct ttg cta atg aca     1399
Ile Ile Gln Ala Val Gly Thr Ala Leu Val Met Pro Leu Leu Met Thr
        120                 125                 130

gtc acg ttg acg gtg gtt cct gcg gag cgg cgt agt tcg atg atg ggc     1447
Val Thr Leu Thr Val Val Pro Ala Glu Arg Arg Ser Ser Met Met Gly
    135                 140                 145

att att tcc atc gtg att tct gtc gcg ccg gcg ctt ggt cct act ttg     1495
Ile Ile Ser Ile Val Ile Ser Val Ala Pro Ala Leu Gly Pro Thr Leu
150                 155                 160                 165

tct ggt gtc att ctt aac tct ttg acc tgg cac tgg ttg ttc tgg atg     1543
Ser Gly Val Ile Leu Asn Ser Leu Thr Trp His Trp Leu Phe Trp Met
            170                 175                 180

atg ctc ccg atc gtg gcc gtt gcg ctg gtt att ggc ttc ttt ttg atc     1591
Met Leu Pro Ile Val Ala Val Ala Leu Val Ile Gly Phe Phe Leu Ile
        185                 190                 195

aaa aat atc ggc gaa acc aag atc acc cca ctg gat gtt ctg tct gtc     1639
Lys Asn Ile Gly Glu Thr Lys Ile Thr Pro Leu Asp Val Leu Ser Val
        200                 205                 210

atc ctt tcg gtg ttt gcc ttc ggt ggt ttg gtg tac gga ttc agt tcc     1687
Ile Leu Ser Val Phe Ala Phe Gly Gly Leu Val Tyr Gly Phe Ser Ser
    215                 220                 225

ttc gga gca atc ctg gag ggc gaa ggc acc gta ggt atc ctc gcg atc     1735
Phe Gly Ala Ile Leu Glu Gly Glu Gly Thr Val Gly Ile Leu Ala Ile
230                 235                 240                 245

gtc gtt ggc gcc atc gca ctt ctc atc ttt gct ttg cga cag cac caa     1783
Val Val Gly Ala Ile Ala Leu Leu Ile Phe Ala Leu Arg Gln His Gln
                250                 255                 260

ctc ggc aaa caa gac aag gca ctg atg gat ctc cgt gct ttc aag gtg     1831
Leu Gly Lys Gln Asp Lys Ala Leu Met Asp Leu Arg Ala Phe Lys Val
            265                 270                 275

agg aac ttc agc ttc tcc ttg acc acc atc ctt ttg gcg ttc ggc gcg     1879
Arg Asn Phe Ser Phe Ser Leu Thr Thr Ile Leu Leu Ala Phe Gly Ala
        280                 285                 290

atg ctc gga acc gtc atg gtt ttg cca atc tac ctg cag act tct ctc     1927
Met Leu Gly Thr Val Met Val Leu Pro Ile Tyr Leu Gln Thr Ser Leu
    295                 300                 305

gga gtt act gct ttg gtg acc ggt ttg gtt gtt atg ccc ggc ggc ctc     1975
Gly Val Thr Ala Leu Val Thr Gly Leu Val Val Met Pro Gly Gly Leu
310                 315                 320                 325

ctc cag ggt ctg atc agc cca ttc atc gga cgt ttc tac gac aag gtt     2023
Leu Gln Gly Leu Ile Ser Pro Phe Ile Gly Arg Phe Tyr Asp Lys Val
                330                 335                 340

ggt cca cgt ccg ttg ctg att ccc gga gca att gcg ctg gct atc gcg     2071
Gly Pro Arg Pro Leu Leu Ile Pro Gly Ala Ile Ala Leu Ala Ile Ala
            345                 350                 355

gca tcc tcg atg act ttc ctc aat gag aat tca cct gtg tgg atg gtc     2119
Ala Ser Ser Met Thr Phe Leu Asn Glu Asn Ser Pro Val Trp Met Val
        360                 365                 370

gtg gtc atg cac gtt gtg ttc agc atc ggc atg tgt ttg atg atg acc     2167
Val Val Met His Val Val Phe Ser Ile Gly Met Cys Leu Met Met Thr
    375                 380                 385

cct ctc atg acc acc gct ctc ggc gcc ctt ccg aag cac ctc tat ggt     2215
Pro Leu Met Thr Thr Ala Leu Gly Ala Leu Pro Lys His Leu Tyr Gly
390                 395                 400                 405

cac ggc tcc gca att ctg aac acg ttc caa cag ctc gca ggc gca gcc     2263
His Gly Ser Ala Ile Leu Asn Thr Phe Gln Gln Leu Ala Gly Ala Ala
                410                 415                 420

gga aca gcg atc atg att gcg gca ctt tcc ttc ggc act tcc att gca     2311
Gly Thr Ala Ile Met Ile Ala Ala Leu Ser Phe Gly Thr Ser Ile Ala
            425                 430                 435

gcg tct tcc gga tct gca cac gcc gaa gct gtc gcc gct ggc acc aag     2359
Ala Ser Ser Gly Ser Ala His Ala Glu Ala Val Ala Ala Gly Thr Lys
        440                 445                 450

gtt gcg ttc atc gca ggc gca atc atc gca gtg atc gct ttg gtt att     2407
Val Ala Phe Ile Ala Gly Ala Ile Ile Ala Val Ile Ala Leu Val Ile
    455                 460                 465

tcc ctc ttc gtc act cgc gtc gag gaa gaa act cac taaacaccaa          2453
Ser Leu Phe Val Thr Arg Val Glu Glu Glu Thr His
470                 475                 480

aaaatggggc agaaggtcat tgactttctg ccccattttt tgcttctcga cgcctcccgc   2513

cgccacctca ccttaagcgt gctcagcgct ccaggttttg ttctcgccac ccggtttgtg   2573

gttcccacac caacagtttg tcaatgaggc ggctttctgg atttttcagc gaaataacaa 2633

ttctgttctg agaagcatat tccacttcaa actgcctttt gggcaagttc acagttgtta 2693

gttgctttgt tgttttagga tcgtggaaag cgtagatatc ttgttgttcg tggaacatca 2753

tgagtgatcc gataggaaat cccagcgttg gagaatcccc tggtggaaga tcggaaagtg 2813

gccaataacc tgcatcggga tttccgtgga cctctacact gaggatttca agatcctgat 2873

taaacgtgat ctggtgaaga tcggaacgca gataaattcg ttcaccgtat tggtaaatcg 2933

tcgaaatctc ggcgtatcca agatcaagct caattgcttt gaagggtttg atcctaagga 2993

tggtttgttt ccaggttccg ttgtcagcgc ttacgacatg gaagttcttc ccgaatcttt 3053

tgtgtagttt ccagcctgcg gggatcgctg ggacagggtc ggttgcctct ttaaggtctg 3113

gaagttccca aagttttttc gctgcgcggg ctctattgcc ggattcgagg gcgatctcat 3173

tgaaggtggg tatggagatc tcgtgtacta gcttgcctgt ggagacattg aaaccgcgga 3233

cgaagggcaa gctcttatcg gacagccaga atgtttcacc gtcacaggta ctggtgctgc 3293

tccaatccaa ggattcttga tatctaggag gtttcgcacc gtcgagggtc acgtcgataa 3353

ttgcgtcctt accgtggcgg tagactgcgg tgatgaccgc tgacgtgcgg cggctcgtgt 3413

ccgcgtagga gctgtgggtg aagtagccgg tga 3446

<210> 12
<211> 481
<212> PRT
<213> Corynebacterium glutamicum ATCC14067

<400> 12

Met Asp Ser Gln Ile Asn Thr Gln Thr Ser Pro Ala Ala Ala Lys Leu
1               5                   10                  15

Pro Arg Glu Val Val Val Val Leu Ser Ile Leu Val Ile Ser Ala Met
            20                  25                  30

Ile Met Ile Leu Asn Glu Thr Ile Leu Ser Val Ala Leu Pro Ser Ile
        35                  40                  45

Met Ala Asp Phe Gln Val Pro Glu Thr Thr Ala Gln Trp Leu Thr Thr
    50                  55                  60

Gly Phe Met Leu Thr Met Ala Val Val Ile Pro Thr Thr Gly Tyr Leu
65                  70                  75                  80

Leu Asp Arg Phe Ser Thr Lys Thr Ile Phe Val Thr Ala Leu Leu Phe
                85                  90                  95

Phe Thr Val Gly Thr Leu Thr Ala Ala Leu Ala Pro Thr Phe Ala Val
            100                 105                 110

Leu Leu Gly Ala Arg Ile Ile Gln Ala Val Gly Thr Ala Leu Val Met
        115                 120                 125

Pro Leu Leu Met Thr Val Thr Leu Thr Val Val Pro Ala Glu Arg Arg
    130                 135                 140

Ser Ser Met Met Gly Ile Ile Ser Ile Val Ile Ser Val Ala Pro Ala
145                 150                 155                 160

Leu Gly Pro Thr Leu Ser Gly Val Ile Leu Asn Ser Leu Thr Trp His
                165                 170                 175

Trp Leu Phe Trp Met Met Leu Pro Ile Val Ala Val Ala Leu Val Ile
            180                 185                 190

Gly Phe Phe Leu Ile Lys Asn Ile Gly Glu Thr Lys Ile Thr Pro Leu
        195                 200                 205

Asp Val Leu Ser Val Ile Leu Ser Val Phe Ala Phe Gly Gly Leu Val
    210                 215                 220

Tyr Gly Phe Ser Ser Phe Gly Ala Ile Leu Glu Gly Glu Gly Thr Val
225                 230                 235                 240

Gly Ile Leu Ala Ile Val Val Gly Ala Ile Ala Leu Leu Ile Phe Ala
                245                 250                 255

Leu Arg Gln His Gln Leu Gly Lys Gln Asp Lys Ala Leu Met Asp Leu
            260                 265                 270

Arg Ala Phe Lys Val Arg Asn Phe Ser Phe Ser Leu Thr Thr Ile Leu
        275                 280                 285

Leu Ala Phe Gly Ala Met Leu Gly Thr Val Met Val Leu Pro Ile Tyr
    290                 295                 300

Leu Gln Thr Ser Leu Gly Val Thr Ala Leu Val Thr Gly Leu Val Val
305                 310                 315                 320

Met Pro Gly Gly Leu Leu Gln Gly Leu Ile Ser Pro Phe Ile Gly Arg
                325                 330                 335

Phe Tyr Asp Lys Val Gly Pro Arg Pro Leu Leu Ile Pro Gly Ala Ile
            340                 345                 350

Ala Leu Ala Ile Ala Ala Ser Ser Met Thr Phe Leu Asn Glu Asn Ser
        355                 360                 365

Pro Val Trp Met Val Val Val Met His Val Val Phe Ser Ile Gly Met
    370                 375                 380

Cys Leu Met Met Thr Pro Leu Met Thr Thr Ala Leu Gly Ala Leu Pro
385                 390                 395                 400

Lys His Leu Tyr Gly His Gly Ser Ala Ile Leu Asn Thr Phe Gln Gln
                405                 410                 415

Leu Ala Gly Ala Ala Gly Thr Ala Ile Met Ile Ala Ala Leu Ser Phe
            420                 425                 430

Gly Thr Ser Ile Ala Ala Ser Ser Gly Ser Ala His Ala Glu Ala Val
        435                 440                 445

Ala Ala Gly Thr Lys Val Ala Phe Ile Ala Gly Ala Ile Ile Ala Val
    450                 455                 460

Ile Ala Leu Val Ile Ser Leu Phe Val Thr Arg Val Glu Glu Glu Thr
465                 470                 475                 480

His

<210>  13
<211>  1585
<212>  DNA
<213>  artificial sequence

<220>
<223>  nucleotide sequence comprising a deletion of the cds of the lmrB
       gene of Corynebacterium glutamicum ATCC13032 and the adjoining
       stop codon accompanied by insertion of the recognition site for
       restriction endonuclease EcoRV

<220>
<221>  misc_feature
<222>  (1)..(788)
<223>  nucleotide sequence of ATCC13032 upstream (5'-flanking sequence)
       of sequence coding for LmrB polypeptide

<220>
<221>  misc_feature
<222>  (789)..(794)
<223>  artificially inserted recognition site for restriction
       endonuclease EcoRV

<220>
<221>  misc_feature

<222>   (795)..(1585)
<223>   nucleotide sequence of ATCC13032 downstream (3'-flanking
        sequence) of sequence coding for LmrB polypeptide

<400>   13
aaggctacgt tcaaaatcct ccaagcattg gccgtccggg atgcgatata aagatcgtta      60

cctctcgtcg cgtgatattc gagaggttca tgggatccga gttacgggag cgttccgcac     120

atttttggac atcgctttgg atgatggggt ggtggcggct gtggtcacta ttgattcagc     180

tcgaagacag aatccatcgc ttacgcgtga gaagttaatg cacagtgcgg aaagtttccc     240

gaggcatcgg ggtgtgaagg cgtatcggca ggcgattgag ttgtcgattc ccaattcgga     300

tagtgctcag gagacgaggg ctcggttaat ccttcgggag gccaagctcc cggaaatcca     360

gtcagtgaag gtgcaggccc gtttcgatca atcgcacaac aagtatttcc tcgtcgattt     420

cttgatcaat gagtggatca tcgtggagat tgatggacgt tcgaaatatg attccccgga     480

gctcaatgag gtgctcatgg ctgaacgcga tcgggagaaa ttcttcctca atcagggcta     540

tgcggtctta agaatcgatc cgaaacagtt agacctcaac caagatgggg agtgtgagtt     600

catcggaatc ctcaaaaaca ctttgcagaa gaccccacct gagcacctca agcaagccgc     660

ctaaacactc caagaaccac catttgcaca atgcacttgc ttgtggcact ctttagtagt     720

tttttctcat agctcagttt cgcaacttta gagaactcta gaaactgagc ttcatgctgt     780

gaaaggccga tatcaatggg gcagaaggtc attgactttc tgccccattt ttttgcttct     840

cgacgcctcc cgccgccacc tcaccttaag cgtgctcagc gctccaggtt ctgttctcgc     900

cacccggttt gtggttccca caccaacagc ttgttggtag gtcggctttc tgggtttttc     960

agtgaaataa caattttggt tcgggaagaa tatgccactg aaaaactatt cctgtctaag    1020

ttcacagttg ttatttgttc tgttgtttca gggtcgtgga aagcatatgt gccttcttgc    1080

tcatgaaaca tcatgagtgt tccggtggga aaaccgagtg ttggagaatc cccaggtgga    1140

agataaatta gaccagattc gggaacctca gggtcgccaa agacttctac gttgagaatc    1200

tcgaggtcct ggttaaacgt gatgtggtgg ttactagcgc gtaagtaaat tcgatcaccg    1260

tatgggtaaa ttgctgtgat tatcgatcgg ccgatatcaa gatctactgc tttaaatggt    1320

ttaagcctaa ggattgtttg aagccaaaag ttgtcattaa gacgagggtt ttcgcgtgtg    1380

acattgtgca agtttttccc gaagctgtct aacaatctcc aacctgctgg aatcgaaggg    1440

acagcttcga atacttcttt aagatccgga agctcccatg tttttccctt gcaatcactg    1500

aaatcgtgag gccattgctt ttgttttacg cgggctctat tagcggaatc gaggacgatc    1560

tcttggaacg tggggatgga gatct                                          1585


<210>   14
<211>   1585
<212>   DNA

<213>  artificial sequence

<220>
<223>  nucleotide sequence comprising a deletion of the cds of the lmrB
      gene of Corynebacterium glutamicum ATCC14067 and the adjoining
      stop codon accompanied by insertion of the recognition site for
      restriction endonuclease EcoRV


<220>
<221>  misc_feature
<222>  (1)..(788)
<223>  nucleotide sequence of ATCC14067 upstream (5'-flanking sequence)
      of sequence coding for LmrB polypeptide

<220>
<221>  misc_feature
<222>  (789)..(794)
<223>  artificially inserted recognition site for restriction
      endonuclease EcoRV

<220>
<221>  misc_feature
<222>  (789)..(794)
<223>  nucleotide sequence of ATCC14067 downstream (3'-flanking
      sequence) of sequence coding for LmrB polypeptide

<400>  14
tgtcaaagac ttcccagtag aaacctcccc actcacacgc caacaccgca ccaagccagg      60

cgtcaccgaa aagtgggacc tctacgtccg cggatttgaa ctagcaaccg gatactccga     120

actcatcgac ccagtcatcc aacgcgaacg cttcgaagac caagcccgcc tcgccgccga     180

cggagacgac gaagccatgg tcctcgacga agacttcctc accgcaatgg aacaaggcat     240

gccaccaacc tccggcaacg gcatgggaat cgaccgcctc ctcatggccc tcaccggcct     300

cggaatccgc gaaaccgtac tcttcccaat ggtgaaacca gaacaaaagt aggttttctg     360

tttgggtagg ttagctccag gactttgtgt cctggagttt tcctcttcct agggttgatc     420

gggatgggtt ttgtaaggtc gggtcggcgt cggtccgtaa gttcgatttc acggtttcga     480

gaccttaaca gcgatttcac gggtttcggt ttctcgcgtt caatttcttg tactcaattt     540

cgcatattcg ataccactag gaaagacgtc aaaatccgtt agacctcgac caaaacggcg     600

agtgcgagtt catcggaatc ctcaaaaaca ctttgcagaa gaccccacca gagcacctca     660

ggcaagccgc ctaaacactc caagaaccac cacttgcaca atgcacttgc ttatggcact     720

ctttaggagt tttttctcat agctcagttt cgtaacacta gaaactgagc ttcatgctgt     780

gaaaggctga tatcaatggg gcagaaggtc attgactttc tgccccattt tttgcttctc     840

gacgcctccc gccgccacct caccttaagc gtgctcagcg ctccaggttt tgttctcgcc     900

acccggtttg tggttcccac accaacagtt tgtcaatgag gcggctttct ggatttttca     960

gcgaaataac aattctgttc tgagaagcat attccacttc aaactgcctt ttgggcaagt    1020

tcacagttgt tagttgcttt gttgttttag gatcgtggaa agcgtagata tcttgttgtt    1080

cgtggaacat catgagtgat ccgataggaa atcccagcgt tggagaatcc cctggtggaa 1140

gatcggaaag tggccaataa cctgcatcgg gatttccgtg gacctctaca ctgaggattt 1200

caagatcctg attaaacgtg atctggtgaa gatcggaacg cagataaatt cgttcaccgt 1260

attggtaaat cgtcgaaatc tcggcgtatc caagatcaag ctcaattgct ttgaagggtt 1320

tgatcctaag gatggtttgt ttccaggttc cgttgtcagc gcttacgaca tggaagttct 1380

tcccgaatct tttgtgtagt ttccagcctg cggggatcgc tgggacaggg tcggttgcct 1440

ctttaaggtc tggaagttcc caaagttttt tcgctgcgcg ggctctattg ccggattcga 1500

gggcgatctc attgaaggtg ggtatggaga tctcgtgtac tagcttgcct gtggagacat 1560

tgaaaccgcg gacgaagggc aagct 1585

<210> 15
<211> 1266
<212> DNA
<213> Corynebacterium glutamicum ATCC13032

<220>
<221> CDS
<222> (1)..(1263)
<223> nucleotide sequence encoding aspartokinase

<220>
<221> misc_feature
<222> (1)..(3)
<223> gtg start codon

<220>
<221> misc_feature
<222> (1264)..(1266)
<223> stop codon

<400> 15
gtg gcc ctg gtc gta cag aaa tat ggc ggt tcc tcg ctt gag agt gcg 48
Met Ala Leu Val Val Gln Lys Tyr Gly Gly Ser Ser Leu Glu Ser Ala
1               5                   10                  15

gaa cgc att aga aac gtc gct gaa cgg atc gtt gcc acc aag aag gct 96
Glu Arg Ile Arg Asn Val Ala Glu Arg Ile Val Ala Thr Lys Lys Ala
            20                  25                  30

gga aat gat gtc gtg gtt gtc tgc tcc gca atg gga gac acc acg gat 144
Gly Asn Asp Val Val Val Val Cys Ser Ala Met Gly Asp Thr Thr Asp
        35                  40                  45

gaa ctt cta gaa ctt gca gcg gca gtg aat ccc gtt ccg cca gct cgt 192
Glu Leu Leu Glu Leu Ala Ala Ala Val Asn Pro Val Pro Pro Ala Arg
    50                  55                  60

gaa atg gat atg ctc ctg act gct ggt gag cgt att tct aac gct ctc 240
Glu Met Asp Met Leu Leu Thr Ala Gly Glu Arg Ile Ser Asn Ala Leu
65                  70                  75                  80

gtc gcc atg gct att gag tcc ctt ggc gca gaa gcc caa tct ttc acg 288

Val Ala Met Ala Ile Glu Ser Leu Gly Ala Glu Ala Gln Ser Phe Thr
                85                  90                  95

ggc tct cag gct ggt gtg ctc acc acc gag cgc cac gga aac gca cgc      336
Gly Ser Gln Ala Gly Val Leu Thr Thr Glu Arg His Gly Asn Ala Arg
            100                 105                 110

att gtt gat gtc act cca ggt cgt gtg cgt gaa gca ctc gat gag ggc      384
Ile Val Asp Val Thr Pro Gly Arg Val Arg Glu Ala Leu Asp Glu Gly
        115                 120                 125

aag atc tgc att gtt gct ggt ttc cag ggt gtt aat aaa gaa acc cgc      432
Lys Ile Cys Ile Val Ala Gly Phe Gln Gly Val Asn Lys Glu Thr Arg
    130                 135                 140

gat gtc acc acg ttg ggt cgt ggt ggt tct gac acc act gca gtt gcg      480
Asp Val Thr Thr Leu Gly Arg Gly Gly Ser Asp Thr Thr Ala Val Ala
145                 150                 155                 160

ttg gca gct gct ttg aac gct gat gtg tgt gag att tac tcg gac gtt      528
Leu Ala Ala Ala Leu Asn Ala Asp Val Cys Glu Ile Tyr Ser Asp Val
                165                 170                 175

gac ggt gtg tat acc gct gac ccg cgc atc gtt cct aat gca cag aag      576
Asp Gly Val Tyr Thr Ala Asp Pro Arg Ile Val Pro Asn Ala Gln Lys
            180                 185                 190

ctg gaa aag ctc agc ttc gaa gaa atg ctg gaa ctt gct gct gtt ggc      624
Leu Glu Lys Leu Ser Phe Glu Glu Met Leu Glu Leu Ala Ala Val Gly
        195                 200                 205

tcc aag att ttg gtg ctg cgc agt gtt gaa tac gct cgt gca ttc aat      672
Ser Lys Ile Leu Val Leu Arg Ser Val Glu Tyr Ala Arg Ala Phe Asn
    210                 215                 220

gtg cca ctt cgc gta cgc tcg tct tat agt aat gat ccc ggc act ttg      720
Val Pro Leu Arg Val Arg Ser Ser Tyr Ser Asn Asp Pro Gly Thr Leu
225                 230                 235                 240

att gcc ggc tct atg gag gat att cct gtg gaa gaa gca gtc ctt acc      768
Ile Ala Gly Ser Met Glu Asp Ile Pro Val Glu Glu Ala Val Leu Thr
                245                 250                 255

ggt gtc gca acc gac aag tcc gaa gcc aaa gta acc gtt ctg ggt att      816
Gly Val Ala Thr Asp Lys Ser Glu Ala Lys Val Thr Val Leu Gly Ile
            260                 265                 270

tcc gat aag cca ggc gag gct gcg aag gtt ttc cgt gcg ttg gct gat      864
Ser Asp Lys Pro Gly Glu Ala Ala Lys Val Phe Arg Ala Leu Ala Asp
        275                 280                 285

gca gaa atc aac att gac atg gtt ctg cag aac gtc tct tct gta gaa      912
Ala Glu Ile Asn Ile Asp Met Val Leu Gln Asn Val Ser Ser Val Glu
    290                 295                 300

gac ggc acc acc gac atc acc ttc acc tgc cct cgt tcc gac ggc cgc      960
Asp Gly Thr Thr Asp Ile Thr Phe Thr Cys Pro Arg Ser Asp Gly Arg
305                 310                 315                 320

cgc gcg atg gag atc ttg aag aag ctt cag gtt cag ggc aac tgg acc     1008
Arg Ala Met Glu Ile Leu Lys Lys Leu Gln Val Gln Gly Asn Trp Thr
                325                 330                 335

aat gtg ctt tac gac gac cag gtc ggc aaa gtc tcc ctc gtg ggt gct 1056
Asn Val Leu Tyr Asp Asp Gln Val Gly Lys Val Ser Leu Val Gly Ala
            340                 345                 350

ggc atg aag tct cac cca ggt gtt acc gca gag ttc atg gaa gct ctg 1104
Gly Met Lys Ser His Pro Gly Val Thr Ala Glu Phe Met Glu Ala Leu
        355                 360                 365

cgc gat gtc aac gtg aac atc gaa ttg att tcc acc tct gag att cgt 1152
Arg Asp Val Asn Val Asn Ile Glu Leu Ile Ser Thr Ser Glu Ile Arg
    370                 375                 380

att tcc gtg ctg atc cgt gaa gat gat ctg gat gct gct gca cgt gca 1200
Ile Ser Val Leu Ile Arg Glu Asp Asp Leu Asp Ala Ala Ala Arg Ala
385                 390                 395                 400

ttg cat gag cag ttc cag ctg ggc ggc gaa gac gaa gcc gtc gtt tat 1248
Leu His Glu Gln Phe Gln Leu Gly Gly Glu Asp Glu Ala Val Val Tyr
                405                 410                 415

gca ggc acc gga cgc taa 1266
Ala Gly Thr Gly Arg
            420

<210> 16
<211> 421
<212> PRT
<213> Corynebacterium glutamicum ATCC13032

<400> 16

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly
        115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190

Leu Glu Lys Leu Ser Phe Glu Glu Met Leu Glu Leu Ala Ala Val Gly
        195                 200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420

201700286
4